# EUROPÄISCHE PATENTSCHRIFT

(11) **EP 3 762 478 B1**
(45) Veröffentlichungstag und Bekanntmachung des Hinweises auf die Patenterteilung: **03.05.2023**
(21) Anmeldenummer: 19700635.6
(22) Anmeldetag: 04.01.2019
(51) Int. Cl.: C12M 1/00, C12M 1/34, C12M 1/36

(54) **ÜBERDRUCKSICHERUNG**
OVERPRESSURE PROTECTION MEANS
PROTECTION CONTRE LA SURPRESSION

(30) Priorität: 04.05.2018 DE 102018003676
(43) Veröffentlichungstag der Anmeldung: 13.01.2021
(73) Patentinhaber: Sartorius Stedim Biotech GmbH, 37079 Göttingen (DE)
(72) Erfinder: DIEL, Bernhard, 37127 Dransfeld (DE); MIGGE, Andreas, 37081 Göttingen (DE); HIELSCHER, Matthias, 37235 Hessisch Lichtenau (DE); ESPACHS, Alexandre, 37079 Göttingen (DE); GREBE, André, 34323 Malsfeld (DE)
(74) Vertreter: Novagraaf International SA
(86) Internationale Anmeldenummer: PCT/EP2019/050128
(87) Internationale Veröffentlichungsnummer: WO 2019/211006

(56) Entgegenhaltungen:
- EP-A1- 3 078 735
- WO-A1-2014/044612
- DE-B3-102016 101 350
- DE-T5-112004 002 636
- US-A1- 2011 207 170

## Beschreibung

Die Anmeldung betrifft ein Verfahren, eine Vorrichtung umfassend einen Einweg-Behälter und eine Überdrucksicherung, sowie ein mit der Vorrichtung ausgestattetes System zum Lagern und/oder Verarbeiten und/oder Prozessieren und/oder Aufbereiten mindestens eines Mediums, sowie zum zumindest teilweisen Auffangen des mindestens einen Mediums, wenn die Überdrucksicherung ausgelöst wird.

Ein Einweg-Behälter kann insbesondere ein Einweg-Beutel bzw. "single-use bag" sein. Ein Einweg-Behälter ist im Wesentlichen ein Behälter, der in der Regel nach seiner Verwendung, insbesondere nach seiner einmaligen Verwendung entsorgt wird. In besonderen Fällen kann ein Einweg-Behälter auch zur mehrmaligen, insbesondere zwei-, drei- oder viermaligen Verwendung ausgelegt sein, jedoch ist er nicht zur dauerhaften Verwendung ausgelegt. Ein Einweg-Behälter kann beispielsweise aus einem Kunststoff, insbesondere aus einem verformbaren synthetischen Material ausgeformt sein. Insbesondere sind die genannten Einweg-Behälter ausgelegt bzw. geeignet, als Bestandteile von Bioprozessanlagen verwendet zu werden. Die Verwendung solcher Einweg-Behälter in der Bioverfahrenstechnik ist besonders vorteilhaft gegenüber herkömmlichen Edelstahlbehältern hinsichtlich der Kosten und deren Flexibilität.

Darüber hinaus ist ein Einweg-Behälter und allgemein ein Einweg-System bevorzugt im Wesentlichen leicht sterilisierbar. Insbesondere liegt ein Einweg-Behälter vor der Verwendung bereits in sterilisiertem Zustand vor, sodass der Verwender dahingehend keine Maßnahmen ergreifen muss. Dies ermöglicht es dem Verwender, das Risiko einer Kontamination mit gefährlichen Stoffen zu reduzieren, insbesondere aufgrund der einmaligen Verwendung und der anschließenden Entsorgung, da eine Reinigung und/oder ein Aufbereitung des Einweg-Behälters, was ein Kontaminationsrisiko birgt, vermieden werden kann bzw. können.

Ein entscheidender Faktor bei der Abwägung zwischen der Verwendung eines Einweg-Behälters und eines Stahlbehälters besteht jedoch in der Sicherheit der Vorgänge. Im Falle eines Überdruckes kann bei einem Einweg-Behälter vergleichsweise leicht zu einem Bruch und/oder Riss und/oder einer Leckage in dem Behälter, insbesondere dessen Behälterwandung kommen. Somit kann es zu einem Auslaufen des Behälters, zu einem Verlust eines Mediums und zu einer Kontamination der Umgebung durch das Medium und/oder des Mediums durch die Umgebung kommen. Die Kontamination kann sich somit einerseits auf die Umgebung beziehen, die mit dem ausgelaufenen und/oder entwichenen Medium kontaminiert werden kann. Insbesondere kann das Medium eine biologisch gefährliche Substanz sein und/oder enthalten, durch dessen unkontrolliertes Entweichen in die Umgebung Verwender gefährlichen Substanzen ausgesetzt sein können. Die Kontamination kann sich aber andererseits auch auf das ausgelaufene und/oder entwichene Medium beziehen, welches durch Stoffe der Umgebung kontaminiert werden kann.

Wenn ein Medium, insbesondere ein fluides Medium, wie ein Gas und/oder eine Flüssigkeit und/oder ein Granulat und/oder ein Pulver, in ein System und/oder einen Behälter geleitet und/oder gefüllt wird, kann ein steigender Druck entstehen, insbesondere, wenn die Befüllung nicht richtig reguliert und/oder das System und/oder der Behälter nicht entsprechend entlüftet wird. Der Druck des Mediums kann dazu führen, dass der Behälter den Höchstdruck bzw. den Berstdruck der Vorrichtung und/oder des Behälters und/oder des Systems übersteigt. Der Höchstdruck bzw. der Berstdruck ist der Druck, ab dem ein Abschnitt des Systems und/oder der Vorrichtung und/oder des Behälters beispielsweise eine Leckage bildet und zumindest ein Teil des Mediums aus dem System und/oder der Vorrichtung und/oder dem Behälter austreten kann.

Einer sogenannte Überdruckbeaufschlagung und/oder einem unkontrollierten Überschreiten eines Höchstdrucks bzw. maximal zulässigen Drucks und/oder einem Berstdruck können beispielsweise Fehlfunktionen eines primären Entlüftungsfilters kausal vorausgehen. Eine Überdruckbeaufschlagung kann beispielsweise durch eine Verstopfung mit einem Kondensat, durch ein Festklemmen bzw. ein Verschließen des primären Entlüftungsfilters, durch einen falsch eingegebenen Wert und/oder einen fehlerhaften Regler und /oder durch ein geschlossenes Ventil und/oder eine Schlauchklemme oder eine anderweitig verschlossene Schlauchleitung ausgelöst werden. Ferner kann eine Pumpengeschwindigkeit zu hoch eingestellt sein und/oder einige andere Eingaben bezüglich ordnungsgemäßer Regulierung, Überwachung und/oder Entlüftung fehlerhaft eingestellt sein, sodass ein unkontrolliert ansteigender Druck erzeugt werden kann. Ein unkontrollierter Überdruck kann infolgedessen Leckagen in dem eingesetzten Schlaumaterial, in Verbindungsstücken und/oder in bzw. an Bauteilen der Vorrichtung verursachen oder diese gar zum Zerbersten bringen. Auch können in der Vorrichtung eingebaute Sensoren beeinträchtigt und insbesondere zerstört werden.

Üblicherweise wird bei Einweg-Systemen bzw. single-use-Systemen (SU Systemen) und/oder Einweg-Behältern der Druck mittels Drucksensoren und Steuereinheiten kontrolliert und/oder gesteuert und/oder geregelt. Der Druck wird demnach mittelbar kontrolliert. Bei einer Fehlfunktion einer Steuereinheit und/oder bei einer falschen Installation und/oder einer fehlerhafter Funktion des Drucksensors, der ausgelegt ist, den Betriebsdruck zu messen und zu überwachen, kann es demnach zu Störungen kommen. Ferner kann ein falsches Signal an die Steuerung übermittelt werden, was auch zu einer unkontrollierten Überschreitung des maximal zulässigen Betriebsdruckes führen kann.

Der maximal zulässige Druck einer Vorrichtung und/oder eines Behälters und/oder eines Systems, der im Umfang dieser Anmeldung auch Berstdruck genannt wird, entspricht dem Wert des Drucks, dem eine Vorrichtung ohne Überdrucksicherung maximal standhalten kann und ab dessen Überschreiten die Vorrichtung zumindest teilweise oder zumindest an einem Abschnitt zerberstet und/oder platzt und/oder bricht und/oder sich eine Leckage bildet.

Der Auslösedruck einer Überdrucksicherung ist der Wert des Drucks, dem eine Überdrucksicherung maximal standhält und ab dessen Überschreiten die Überdrucksicherung ausgelöst wird bzw. auslöst. Der Auslösedruck ist bevorzugt im Wesentlichen geringer als der maximal zulässige Druck an der Stelle, an der die Überdrucksicherung an der Vorrichtung angeordnet und/oder eingebaut und/oder integriert ist. Der Auslösedruck sollte jedoch nicht sehr viel geringer sein als der maximal zulässige Druck.

EP 3 078 735 A1 offenbart einen Einwegbehälter zur Vermeidung von Überdruck. Der Einwegbehälter umfasst ein Gehäuse für ein Fluid und eine Überdruckentlastungsvorrichtung, die mit dem Gehäuse fluidisch verbunden ist, wobei die Überdruckentlastungsvorrichtung konfiguriert ist, um Druck aus dem Gehäuse zu lassen, wenn ein Druck des Fluids innerhalb des Gehäuses einen Wert überschreitet, der dem spezifizierten Entlastungsdruck entspricht, wobei der spezifizierte Entlastungsdruck niedriger als der Berstdruck des Gehäuses ist und wobei die Überdruckentlastungsvorrichtung mit einem gesteuerten Weg verbunden ist, um das Fluid auf kontrollierte Weise aus dem Gehäuse zu entlasten.

Der vorliegenden Erfindung liegt entsprechend die Aufgabe zugrunde, eine Vorrichtung mit Einweg-Behälter zur zumindest teilweisen Lagerung und/oder Verarbeitung von mindestens einem Medium mit einer verbesserten Sicherheit bereitzustellen. Diese Aufgabe wird durch die unabhängigen Ansprüche gelöst. Die Gegenstände der abhängigen Ansprüche stellen bevorzugte Ausführungsformen dar.

Die Erfindung betrifft ein System gemäß Anspruch 1 zum Lagern und/oder Verarbeiten und/oder Prozessieren und/oder Aufbereiten mindestens eines Mediums, insbesondere eine Bioprozesseinrichtung, umfassend
- mindestens einen Einweg-Behälter (1), der ausgelegt ist, das mindestens eine Medium (11) zumindest teilweise aufzunehmen; und- mindestens eine Überdrucksicherung (5), welche fluidisch mit dem mindestens einem Einweg-Behälter (1) verbunden ist, wobei die mindestens eine Überdrucksicherung (5) ausgelegt ist, bei einer Auslösung, das mindestens eine Medium (11) zumindest teilweise- in einen bezüglich einer Stromrichtung (F) der Überdrucksicherung (5) vorgelagerten Vorrichtungsbereich (3a') zu führen, und/oder- in den mindestens einen Einweg-Behälter (1) zu führen; und/oder- in mindestens einen weiteren zweiten Behälter (1 a) zu führen,wobei das System ferner umfasst:- eine Hauptleitung (3a) zur fluidischen Verbindung des mindestens einen Einweg-Behälters (1) mit einem Ziel-Behälter (1 b);- mindestens eine Ableitung (3c), die ausgelegt ist, bei der Auslösung der mindestens einen Überdrucksicherung (5) das mindestens eine Medium (11) zumindest teilweise in mindestens einen weiteren Behälter (1a) zu leiten; und/oder- eine Bypass-Leitung (3b), die ausgelegt ist, bei der Auslösung der mindestens einen Überdrucksicherung (5) das mindestens eine Medium (11) zumindest teilweise in den bezüglich einer Stromrichtung (F) der Überdrucksicherung (5) vorgelagerten Vorrichtungsbereich (3a'), insbesondere einen Abschnitt der Hauptleitung und/oder in den mindestens einen Einweg-Behälter (1) zurück zu leiten,- wobei die mindestens eine Überdrucksicherung (5) an der Ableitung (3c) und/oder der Bypass-Leitung (3b) angeordnet ist und bei der Auslösung der mindestens einen Überdrucksicherung (5) die Ableitung (3c) und/oder die Bypass-Leitung (3b) fluidisch mit der Hauptleitung (3a) verbunden ist,- mindestens ein Druck erzeugendes Mittel (4), insbesondere eine Pumpe, wobei das mindestens eine Druck erzeugende Mittel (4) ausgelegt ist, zumindest einen Teil des mindestens einen Mediums von dem mindestens einen Einweg-Behälter (1) in den dritten Behälter (1b) entlang der Flussrichtung (F) zu pumpen,wobei das mindestens eine Druck erzeugende Mittel (4) an dem Einweg-Behälter (1) angeordnet ist und ausgelegt ist, ein Druckgefälle innerhalb des Einweg-Behälters (1) und/oder entlang der Hauptleitung (3a) zu erzeugen.

In anderen Worten, umfasst eine Vorrichtung einen oder mehrere Einweg-Behälter und eine oder mehrere Überdrucksicherungen. Der mindestens eine Einweg-Behälter ist dabei fluidisch mit der mindestens einen Überdrucksicherung verbunden. Beispielsweise können der Einweg-Behälter und die Überdrucksicherung Bestandteile eines einzigen geschlossenen, insbesondere hermetisch geschlossenen (bzw. von einer Umgebung fluidisch getrennten) Systems sein. Die zwei räumlichen Bereiche, in denen sich beiden Bestandteile befinden, können dann bezüglich des Drucks und/oder fluidisch miteinander kommunizieren. Beispielsweise kann ein Druck zwischen beiden räumlichen Bereichen ausgeglichen werden. Die Überdrucksicherung kann, insbesondere durch Überschreiten eines spezifischen (vorbestimmten bzw. vorbestimmbaren) Auslösedrucks ausgelöst werden. Das heißt, dass der Druck in der Vorrichtung und insbesondere unmittelbar an der Überdrucksicherung einen Druck übersteigt, der größer ist als der spezifische Auslösedruck, und zwar der maximal mögliche Druck, dem eine Überdrucksicherung standhält. Nachdem die Überdrucksicherung ausgelöst wurde, wird zumindest ein Teil des zumindest einen Mediums, insbesondere der Teil des zumindest einen Mediums, welcher durch die ausgelöst Überdrucksicherung hindurchtritt, ab- und/oder zurückgeführt in jeweils einen Auffangbehälter und/oder in einen bezüglich einer Stromrichtung der Überdrucksicherung vorgelagerten Vorrichtungsbereich, insbesondere in den mindestens einen Einweg-Behälter. Einen bezüglich einer Stromrichtung der Überdrucksicherung vorgelagerten Vorrichtungsbereich kann beispielsweise ein Teil einer Leitung sein, durch welche das zumindest eine Medium hindurchtritt bzw. hindurchtreten kann, bevor es durch die ausgelöste Überdrucksicherung hindurchtritt. Die ausgelöste Überdrucksicherung bildet insbesondere eine oder mehrere Öffnungen und/oder die ausgelöste Überdrucksicherung wird permeabel für zumindest einen Teil des zumindest einen Mediums. In anderen Worten kann eine Berstscheibe, die auch als Einmal-Membran bekannt ist, zerbersten und eine Öffnung bilden und/oder eine Membran kann permeabel werden wenn ein Auslösedruck überschritten wird.

Der Einweg-Behälter und die Überdrucksicherung müssen jedoch nicht zwingend ein geschlossenes System miteinander bilden. Sie können auch ein im Wesentlichen offenes System bilden, das zumindest teilweise offen zur Umgebung ist. Auch ist eine permanente und dauerhafte offene Verbindung zwischen zwei räumlichen Bereichen, in denen sich jeweils der Einweg-Behälter und die Überdrucksicherung befinden und die bezüglich des Drucks miteinander kommunizieren können, nicht unter jedem Umstand zwingend notwendig. Die räumlichen Bereiche können auch zeitweise durch eine Barriere, beispielsweise einen verstopften Filter und/oder durch eine Pumpe voneinander im Wesentlichen abgeschirmt bzw. getrennt sein.

Insbesondere kann die Vorrichtung einen, zwei, drei, vier, fünf, oder mehr Einweg-Behälter umfassen. Die Vorrichtung kann ferner eine, zwei, drei, vier, fünf, sechs, oder mehr Überdrucksicherungen umfassen. Eine Vorrichtung kann ferner eine, zwei, drei, vier, fünf, sechs, oder mehr Ableitungen und/oder eine, zwei, drei, vier, fünf, sechs, oder mehr Bypass-Leitungen aufweisen bzw. umfassen und/oder an den jeweiligen Ableitungen und/oder Bypass-Leitungen angeschlossen sein oder angeschlossen werden. Die Ableitungen und/oder Bypass-Leitungen können jeweils mittels ein und derselben oder mittels mehrerer Überdrucksicherungen mit einem oder mehreren anderen Bereichen der Vorrichtung fluidisch verbunden sein oder werden. Die Stromrichtung bzw. die wesentliche Flussrichtung zumindest eines Teils des zumindest einen Mediums in mindestens einem Teil und/oder Abschnitt der Vorrichtung kann beispielsweise durch ein Druck erzeugendes Mittel, insbesondere eine Pumpe und/oder eine Wärmequelle, und/oder eine mechanische Presse und/oder ein Gefälle, sowie eine Gewichtskraft zumindest eines Teils des Mediums hervorgerufen werden. Mit dem Begriff "Druck erzeugendes Mittel" sei auch explizit die inhärente Gewichtskraft zumindest eines Teils des zumindest einen Mediums gemeint. Entsprechend kann ein Druck erzeugendes Mittel mehrere Druck erzeugende Mittel umfassen.

Die beschriebene Vorrichtung kann das unkontrollierte Entweichen von Material verhindern, wenn in single-use Prozesslösungen und/oder Vorrichtungen mit mindestens einen Einweg-Behälter beispielsweise durch ein verwendetes Druck erzeugendes Mittel ein Überdruck erzeugt wird, der im Grenzfall Leckagen und/oder Brüche verursacht bzw. verursachen kann. Somit kann ein Verlust zumindest eines Teils des Mediums verhindert werden.

Es kann ferner verhindert werden, dass ein Druck innerhalb zumindest eines Abschnitts der Vorrichtung bei Überschreitung eines speziellen (vorbestimmten bzw. vorbestimmbaren) Auslösedrucks und/oder eines maximal zulässigen Drucks zumindest nicht weiter ansteigt, da zumindest ein Teil des zumindest einen Mediums im Wesentlichen kontrolliert ab- und/oder zurück in einen Auffangbehälter und/oder den Einweg-Behälter geführt wird. Ferner kann insbesondere einem Gas nach dem Auslösen einer Überdrucksicherung ein vergrößertes Volumen bereitgestellt sein, welches es dem Gas erlaubt, sich auszudehnen, um somit den Druck zu senken oder zumindest auf einen vorbestimmten Wert einzuschränken. Der genannte Vorgang, insbesondere das Auslösen und das Öffnen der Überdrucksicherung, geschieht bevorzugt unmittelbar, insbesondere mechanisch, d.h. im Wesentlichen ohne Steuerung durch Signale und/oder Übermittlung von Signalen, beispielsweise von elektrischen Signalen. Die Überdrucksicherung stellt entsprechend zumindest eine Sollbruchstelle dar, die ab einem Auslösedruck bzw. bei Überschreiten eines Auslösedrucks eine Öffnung zum kontrollierten Entweichen zumindest eines Teils des Mediums bildet. Die Überdrucksicherung kann beispielsweise entsprechend einem speziellen Auslösedruck ausgesucht werden. Zusätzlich oder alternativ kann die Überdrucksicherung auf einen gewünschten Auslösedruck eingestellt werden oder nach einem gewünschten Auslösedruck ausgewählt werden. Dabei entspricht der gewünschte Auslösedruck dem Druck in der Vorrichtung, ab dessen Überschreiten, insbesondere der Druck in dem Einweg-Behälter zumindest nicht weiter steigen soll und die Überdrucksicherung ausgelöst werden soll. Beispielsweise können der spezielle Auslösedruck der Überdrucksicherung und/oder der maximal zulässige Druck der Vorrichtung einstellbar sein und bevorzugt vom Verwender je nach Bedarf eingestellt werden können.

Es kann zudem auch eine Kontamination des Mediums durch Stoffe in der Umgebung und/oder eine Kontamination der Umgebung durch Stoffe in dem Medium mittels der beschriebenen Vorrichtung verhindert werden. Beispielsweise kann verhindert werden, dass ein Stoff des zumindest einen Mediums, beispielsweise ein Zellkulturmedium unkontrolliert aus der Vorrichtung austritt und infolgedessen durch Stoffe in der Umgebung kontaminiert wird. Dies ist insbesondere dann der Fall, wenn die Vorrichtung ein steriles und geschlossenes System bildet und zumindest ein Teil des zumindest einen Mediums im Wesentlichen innerhalb des geschlossenen Systems entweder beispielsweise in den Einweg-Behälter zurückgeführt wird und/oder in einen Auffangbehälter geleitet wird.

Ferner ist die beschriebene Vorrichtung besonders vorteilhaft, wenn das Anwendungsrisiko erhöht ist, und zwar dann, wenn beispielsweise toxische, infektiöse und/oder aggressive Stoffe und/oder Gefahrstoffe in dem zumindest einen Medium enthalten sind und prozessiert werden sollen.

Darüber hinaus kann die beschriebene Vorrichtung vorteilhaft verhindern, dass hochwertige und/oder seltene und/oder wertvolle Stoffe, die in dem zumindest einen Medium innerhalb der Vorrichtung enthalten sein können, bei einem Überdruck und der drohenden Gefahr eines möglichen Zerberstens und/oder Zerbrechens und/oder Zerplatzens eines Bestandteils der Vorrichtung verloren geht. Insbesondere in der Pharma-Industrie besteht ein großes Interesse an der Risikominimierung während des Prozessierens, da pharmazeutische Wirkstoffe häufig von erheblichem Wert sein können.

Im Allgemeinen kann eine austretende Flüssigkeit unter Druck in einen Behälter, insbesondere in ein Vorlagegefäß geführt werden, so dass ein eingesetztes Prozessfluid nicht verworfen werden muss, sondern weiter aufbereitet werden kann.

Mittels der Vorrichtung kann demnach eine höhere Prozesssicherheit erzielt werden. Neben üblichen steuerungstechnischen Mitteln, wie der Prozesskontrolle und der Prozessabsicherung, z.B. umfassend Einweg-Drucksensoren, kann die Vorrichtung demnach die Sicherheit bei der Verarbeitung und/oder Prozessierung und/oder Lagerung gefährlicher und/oder schädlicher und/oder empfindlicher und/oder wertvoller Stoffe zusätzlich optimieren oder zumindest verbessern.

Ein weiterer Vorteil kann darin bestehen, dass auf den Einbau von Drucksensoren in einigen Fällen gänzlich verzichtet werden kann, sodass eine vereinfachte und/oder kostenreduzierte Vorrichtung bereitgestellt werden kann. Darüber hinaus kann infolgedessen auch auf die unter Umständen aufwendige Wartung solcher Drucksensoren verzichtet werden, was zu einer weiteren Vereinfachung und Kostenreduktion führen kann. Insbesondere kann sich entsprechend die Bedienung der Vorrichtung durch den Verwender als unkompliziert und vereinfacht erweisen.

Gemäß einem Aspekt ist die mindestens eine Überdrucksicherung unmittelbar in einer Behälterwandung des mindestens einen Einweg-Behälters integriert, insbesondere schließt die Überdrucksicherung im Wesentlichen formschlüssig mit der Behälterwandung ab.

Eine besonders einfache und vorteilhafte Handhabung ist dann gegeben, wenn die Überdrucksicherung in einem möglichst flachen Abschnitt einer Behälterwandung des Einweg-Behälters integriert ist, sodass beispielsweise keine im Wesentlichen abstehenden Teile beim Transport und/oder bei der Verwendung stören oder beschädigt werden können.

Es kann zudem verhindert werden, dass ein Verwender die Bestandteile aus Überdrucksicherung und Einweg-Behälter vor der Verwendung zusammenbauen muss, wenn diese Bestandteile bereits bei der Fertigung beim Hersteller miteinander verbunden werden. Dies kann insbesondere dann der Fall sein, wenn eine Berstscheibe, insbesondere im Wesentlichen formschlüssig in die Behälterwandung integriert wird.

Gemäß einem Aspekt umfasst die mindestens eine Überdrucksicherung eine Einweg-Überdrucksicherung, insbesondere eine mechanische Einweg-Überdrucksicherung, die bevorzugt zumindest teilweise aus einem Edelstahl und/oder einem Kunststoff ausgeformt ist.

Alle Vorteile der Verwendung von Einweg-Bauteilen treffen zu, wenn eine Einweg-Überdrucksicherung zur einmaligen Verwendung zum Einsatz kommt. Der Begriff "einmalige Verwendung" betrifft im Fall einer Einweg-Überdrucksicherung, das einmalige Auslösen. Nach einem einmaligen Auslösen kann die Einweg-Überdrucksicherung entsorgt werden. Falls die Einweg-Überdrucksicherung im Wesentlichen fest verbaut mit einer Einweg-Vorrichtung und/oder einem Einweg-Behälter und/oder darin integriert ist, kann sich der Begriff "einmalige Verwendung" auch auf die einmalige Verwendung der Einweg-Vorrichtung und/oder des Einweg-Behälters richten, ohne dass die Einweg-Überdrucksicherung ausgelöst werden muss. Beispielsweise kann die gesamte Vorrichtung, sofern alle Teile zur einmaligen Verwendung ausgelegt sind, nach ihrer einmaligen Verwendung als Ganzes entsorgt werden. In dem Fall kann vermieden werden, dass es zu einer Kontamination während eines vollständigen oder teilweisen Aufbereitens und/oder der Reinigung der Vorrichtung kommt. Ferner erweist sich die Verwendung von Einweg-Bauteilen als besonders komfortabel. Falls im Wesentlichen relativ günstige Werkstoffe in der Vorrichtung verarbeitet wurden, kann sich die Entsorgung nach einmaliger Verwendung auch als kostengünstig gegenüber der Mehrfach-Verwendung mit Aufbereitung und Reinigung erweisen.

Gemäß einem Aspekt umfasst die mindestens eine Überdrucksicherung mindestens eine aus den Folgenden, ohne darauf beschränkt zu sein: eine Berstscheibe, ein Überströmventil, ein Sicherheitsventil, eine Membran und insbesondere einen elektrischen und/oder mechnischen Kraftbegrenzer einer Pumpe.

Die genannten Überdrucksicherungen, insbesondere eine Berstscheibe, ein Überströmventil, ein Sicherheitsventil, ein Quetschventil und/oder eine Membran erweisen sich als besonders einfache, leicht sterilisierbare und relativ kostengünstige Bauteile. Die Verwendung und/oder Bereitstellung dieser Überdrucksicherungen erfordert keine Handlung und/oder kein wesentliches Wissen durch den Verwender. Die jeweilige Technologie, auf der die Umsetzung einer Berstscheibe, eines Überströmventils, eines Sicherheitsventils, einer Membran und eines Quetschventils basiert, ist einfach und zuverlässig. Insbesondere kann ein Druck an mindestens einem Abschnitt der Vorrichtung unmittelbar durch die genannten Überdrucksicherungen auf einen vorbestimmten Wert limitiert werden. Dabei erfolgt das Auslösen der jeweiligen Überdrucksicherungen im Wesentlichen mechanisch und insbesondere vollständig mechanisch. Mit "mechanisch" ist insbesondere gemeint, dass lediglich der Druck, der einen Maximalwert bzw. einen speziellen (vorbestimmten bzw. vorbestimmbaren) Auslösedruck übersteigt, genügt, die Überdrucksicherung auszulösen. Es muss entsprechend nicht notwendigerweise ein Signal, beispielsweise ein elektrisches und/oder optisches oder ähnliches Signal von einem Sensor an die Überdrucksicherung gesendet werden, um diese auszulösen. Diese Ausführungsform stellt deshalb vorteilhaft eine weitere Vorrichtung mit erhöhter Sicherheit dar.

Gemäß einem Aspekt umfasst die Vorrichtung ferner mindestens eine Ableitung, die dazu ausgelegt ist, bei der Auslösung der mindestens einen Überdrucksicherung das mindestens eine Medium zumindest teilweise in den mindestens einen Einweg-Behälter und/oder in mindestens einen zweiten Behälter zu leiten. Bevorzugt ist die Ableitung in oder an der Überdrucksicherung integriert und/oder angebracht, insbesondere dann, wenn die Überdrucksicherung bereits in oder an der Behälterwandung integriert und/oder angebracht ist. Optional kann die Ableitung einstückig mit der Überdrucksicherung ausgebildet sein. Auch kann die Ableitung einstückig mit der Überdrucksicherung und der Behälterwandung des Einweg-Behälters ausgebildet sein.

Die Ableitung erlaubt es dem Verwender in definierter Weise, ein übertretendes bzw. ein durch eine ausgelöste Überdrucksicherung austretendes Medium abzuleiten. Beispielsweise kann der betroffene Bestandteil des mindestens einen Mediums in einen weiteren Behälter abgeführt und/oder abgeleitet werden. Für den Fall, dass die genannten Bestandteile einstückig miteinander ausgeformt sind, besteht ein besonderer Vorteil darin, dass Kontaktstellen zwischen den Bestandteilen vermieden werden können. Solche Kontaktstellen könnten undicht sein oder undicht werden, wodurch es von außen oder von der Innenseite der Vorrichtung zu Kontaminationen kommen könnte. Deshalb stellt diese Ausführungsform eine weitere Vorrichtung mit erhöhter Sicherheit dar.

Ferner ist die Verwendung für den Verwender besonders einfach und komfortabel, da er möglicherweise keine oder weniger Maßnahmen zur Montage ergreifen muss. Darüber hinaus kann auch in der Herstellung auf die Montage verzichtet werden, was die Herstellung effizienter und entsprechend kostengünstiger gestalten kann.

Gemäß einem Aspekt wird die mindestens eine Überdrucksicherung durch ein elektrisches Signal, insbesondere von mindestens einem Sensor ausgelöst. In einer Ausführungsform kann eine Überdrucksicherung rein mechanisch ausgelöst werden, wohingegen eine weitere Überdrucksicherung mittels eines elektrischen Signals ausgelöst werden kann. Zusätzlich kann eine Überdrucksicherung, die mechanisch ausgelöst werden kann, auch elektrisch ausgelöst werden.

In anderen Worten kann optional eine Überdrucksicherung unmittelbar durch Überschreiten eines Auslösedrucks oder durch ein elektrisches Signal, beispielsweise eines Druck- und/oder Temperatursensors, ausgelöst werden. Ein Druck- und/oder Temperatursensor kann dazu ein elektrisches Signal an die Überdrucksicherung senden und deren Auslösen triggern. Zusätzlich oder alternativ kann eine zweite Überdrucksicherung durch ein elektrisches Signal einer ersten Überdrucksicherung ausgelöst werden, wenn die erste Überdrucksicherung ausgelöst wird. Oder aber die zweite Überdrucksicherung wird gänzlich mittels eines elektrischen Signals ausgelöst, während die erste Überdrucksicherung mechanisch und/oder elektrisch ausgelöst wird. Neben einer ersten und einer zweiten Überdrucksicherung können weitere Überdrucksicherungen bereitgestellt sein, welche in allen denkbaren Variationen ausgelöst werden können.

Wenn eine Überdrucksicherung fehlerhaft hergestellt wurde und beispielsweise durch den vorbestimmten Auslösedruck nicht ausgelöst werden kann, oder wenn fälschlicherweise eine "falsche" Überdrucksicherung bereitgestellt wird, die ab einem höheren Auslösedruck als dem maximal zulässigen Druck ausgelöst wird, so kann die Überdrucksicherung zusätzlich durch ein elektrisches Signal, beispielsweise ausgesendet von einem Drucksensor, ausgelöst werden, um einen weiteren Sicherheitsgrad zu erreichen. Zusätzlich oder optional kann das Signal auch von einem Temperatur- und/oder einem Gas- und/oder einem optischen Sensor an die Überdrucksicherung gesendet werden. Beispielsweise kann im Fall eines Erreichens einer vorbestimmten bzw. vorbestimmbaren Maximaltemperatur zumindest eines Teils des Mediums ein elektrisches Signal zur Auslösung der Überdrucksicherung gesendet werden, sodass ein Medium mit einer zu hohen Temperatur abgeführt wird und dadurch im Wesentlichen verhindert wird, dass dieser Teil des Mediums mit zu hoher Temperatur möglicherweise temperatursensitive Elemente der Vorrichtung und/oder andere Medien darin beschädigt und/oder beeinträchtigt.

Falls eine Vielzahl von (zwei, drei oder mehreren) Überdrucksicherungen bereitgestellt werden, kann es von Vorteil sein, dass, wenn eine Überdrucksicherung mechanisch durch Überschreiten eines Auslösedrucks ausgelöst wird, die anderen Überdrucksicherungen, an denen der lokale (Partial-)Druck möglicherweise noch nicht den jeweiligen maximalen Wert bzw. Druck erreicht und insbesondere überstiegen hat, elektrisch ausgelöst werden. Derart können weitere Öffnungen erzeugt werden, durch welche sich zumindest ein Teil des zumindest einen Mediums, beispielsweise ein Fluid (insbesondere ein Gas) ausdehnen kann und kontrolliert entweichen und/oder kontrolliert abgeführt und/oder aufgefangen werden kann. In dem Fall kann im Voraus vermieden werden, dass es in verschiedenen Abschnitten einer Vorrichtung zu einem ansteigenden Druck kommt nach Auslösung der jeweiligen Überdrucksicherungen. Somit kann die Sicherheit der Vorrichtung zusätzlich verbessert werden. Diese Ausführungsform stellt demnach eine weitere Vorrichtung mit erhöhter Sicherheit dar.

Gemäß einem Aspekt kann eine Überdrucksicherung bei der Auslösung ein Signal an einen anderen Bestandteil der Vorrichtung versenden. Insbesondere kann eine Überdrucksicherung bei deren Auslösung ein elektrisches Signal an eine Pumpe senden, welches die Pumpe dazu veranlasst, abzuschalten. Alternativ oder zusätzlich kann eine Überdrucksicherung bei deren Auslösung ein Signal an mindestens eine andere Sicherungseinheit versenden und diese dazu veranlassen, eine Funktion abzuschalten oder auszulösen. Beispielsweise kann die Hauptsicherung einer Stromversorgung durch eine Überdrucksicherung bei deren Auslösung ein Signal erhalten, welches die Hauptsicherung dazu veranlasst, die Stromversorgung abzuschalten.

Eine Rückkopplung zwischen einer Überdrucksicherung und einem anderen Element, beispielsweise einer anderen Sicherung und/oder einem Motor und/oder einer Pumpe hat den Vorteil, dass zumindest Teile des Systems in einer Gefahrenlage komplett abgeschaltet werden können, wenn eine Überdrucksicherung einmal ausgelöst wurde. Dies kann verhindern, dass weitere Teile des zumindest einen Mediums durch die geöffnete Überdrucksicherung abgeführt werden und/oder austreten und möglicherweise infolgedessen ein Auffangbehälter überläuft. Insbesondere kann verhindert werden, dass es zu einem weiteren Ansteigen des Drucks auch in anderen Abschnitten der Vorrichtung kommt. Diese Ausführungsform stellt demnach auch eine weitere Vorrichtung mit erhöhter Sicherheit dar.

Die Erfindung betrifft ferner ein System umfassend:
- mindestens einen Einweg-Behälter, der ausgelegt ist, das mindestens eine Medium zumindest teilweise aufzunehmen;
- mindestens eine Überdrucksicherung, welche fluidisch mit dem mindestens einem Einweg-Behälter verbunden ist;
- eine Hauptleitung zur fluidischen Verbindung des mindestens einen Einweg-Behälters mit einem Ziel-Behälter; und
- mindestens eine Ableitung, die ausgelegt ist, bei der Auslösung der mindestens einen Überdrucksicherung das mindestens eine Medium zumindest teilweise in mindestens einen weiteren Behälter zu leiten; und/oder
- eine Bypass-Leitung, die ausgelegt ist, bei der Auslösung der mindestens einen Überdrucksicherung das mindestens eine Medium zumindest teilweise in den bezüglich einer Stromrichtung der Überdrucksicherung vorgelagerten Vorrichtungsbereich, insbesondere einen Abschnitt der Hauptleitung und/oder in den mindestens einen Einweg-Behälter zurück zu leiten,
wobei die mindestens eine Überdrucksicherung an der Ableitung und/oder der Bypass-Leitung angeordnet ist und bei der Auslösung der mindestens einen Überdrucksicherung die Ableitung und/oder die Bypass-Leitung fluidisch mit der Hauptleitung verbunden ist.

In anderen Worten handelt es sich um ein System umfassend eine der zuvor genannten Vorrichtungen, insbesondere nach einer bevorzugten Ausführungsform, wobei das System ferner umfasst:
- eine Hauptleitung zur fluidischen Verbindung des mindestens einen Einweg-Behälters mit einem zusätzlichen dritten Behälter, der einem Ziel-Behälter entspricht bzw. der ein Zielbehälter ist; und
- mindestens eine Ableitung, die ausgelegt ist, bei der Auslösung der mindestens einen Überdrucksicherung das mindestens eine Medium zumindest teilweise in den mindestens einen zusätzlichen zweiten Behälter zu leiten; und/oder
- eine Bypass-Leitung, die ausgelegt ist, bei der Auslösung der mindestens einen Überdrucksicherung das mindestens eine Medium zumindest teilweise in den bezüglich einer Stromrichtung der Überdrucksicherung vorgelagerten Vorrichtungsbereich, insbesondere einen Abschnitt der Hauptleitung und/oder in den mindestens einen Einweg-Behälter zurück zu leiten,
wobei die mindestens eine Überdrucksicherung an der Ableitung und/oder der Bypass-Leitung angeordnet ist und bei der Auslösung der mindestens einen Überdrucksicherung die Ableitung und/oder die Bypass-Leitung fluidisch mit der Hauptleitung verbunden ist.

Insbesondere bilden Teile des Systems bzw. Volumina des Systems, insbesondere Behälter und/oder Leitungen und/oder Ableitungen und/oder Bypass-Leitungen nach dem Auslösen einer Überdrucksicherung physikalische kommunizierende Röhren, welche ein Fluid und einen Druck austauschen können, sofern eine fluidische Verbindung zwischen den entsprechenden Teilen bzw. Abschnitten besteht.

Neben den Vorteilen der Ausführungsformen einer Vorrichtung hat das beschriebene System weitere Vorteile, die in seiner Gesamtheit aber auch in seinen einzelnen Bestandteilen liegen. Das Bereitstellen einer Hauptleitung erlaubt dem Verwender, einen Teil des Mediums, das einen Prozess und/oder einen Vorgang durchlaufen hat, räumlich von dem Teil des Mediums, dem dieser Prozess und/oder Vorgang noch bevorsteht, zu trennen.

Das Bereitstellen einer Ableitung und/oder einer Bypass-Leitung hat den Vorteil, dass ein Medium, welches nach der Auslösung einer Überdrucksicherung durch selbige hindurchtritt, definiert und kontrolliert abgeleitet und/oder in einen bezüglich einer Stromrichtung der Überdrucksicherung vorgelagerten Bereich zurückgeführt werden kann. Eine definierte und/oder kontrollierte Ab- bzw. Zurückleitung zumindest eines Teils des Mediums besteht in dem Verlauf, den das Medium zwischen der Überdrucksicherung und dem Zielvolumen nimmt. Beispielsweise kann das Medium zumindest teilweise durch einen Schlauch und/oder ein Rohr geleitet werden. Ferner kann definiert und/oder kontrolliert werden, ob das Teilsystem, in das und/oder durch welches zumindest ein Teil des Mediums geführt wird, ein im Wesentlichen geschlossenes oder ein im Wesentlichen offenes System darstellen soll. Ferner können sämtliche physikalische Parameter, wie Volumen, Druck, Temperatur etc. definiert und/oder kontrolliert werden.

Das Bereitstellen einer Ableitung und/oder einer Bypass-Leitung kann den Vorteil haben, dass je nach Bedarf ein geeignetes Zielvolumen ausgewählt werden kann. Beispielsweise kann das Zielvolumen für einen mittels einer Ableitung abgeleiteten Teil des Mediums in einem Auffangbehälter bestehen. Das Zielvolumen kann für ein mittels einer Bypass-Leitung zurückgeführten Teil des Mediums in dem bezüglich einer Stromrichtung der Überdrucksicherung vorgelagerten Bereich, insbesondere in dem Einweg-Behälter bestehen.

Gemäß einem Aspekt umfasst das System den weiteren dritten Behälter, der ausgelegt ist, das mindestens eine Medium zumindest teilweise aufzunehmen und der bevorzugt ein Einweg-Behälter ist.

Ein Vorteil, einen weiteren Behälter zusätzlich zum Einweg-Behälter und gegebenenfalls zu einem Auffangbehälter bereitzustellen, liegt darin, dass ein Medium, das sich zumindest teilweise in dem Einweg-Behälter befindet, in den weiteren Behälter überführt werden kann, wenn beispielsweise ein chemischer und/oder biologischer Prozess in dem Einweg-Behälter abgelaufen ist. Beispielsweise kann das Medium auf seinem Weg zu dem weiteren Behälter durch einen Filter gelangen, wo es zumindest teilweise aufgereinigt und/oder gefiltert wird, bevor es in den weiteren Behälter gelangt. Auf diese Weise kann ein Teil des Mediums, das einen Prozess und/oder einen Vorgang durchlaufen hat, räumlich von dem Teil des Mediums getrennt werden, dem dieser Prozess und/oder Vorgang noch bevorsteht.

Das Bereitstellen eines weiteren Einweg-Behälters hat den Vorteil, dass ein weiterer Bestandteil des Systems nach einmaliger Verwendung entsorgt werden kann. Die Vorteile der Bereitstellung von Einweg-Behältern wurden bereits zuvor dargelegt.

Gemäß einem Aspekt umfasst das System mindestens ein Druck erzeugendes Mittel, insbesondere eine Pumpe, wobei das mindestens eine Druck erzeugende Mittel ausgelegt ist, zumindest einen Teil des mindestens einen Mediums von dem mindestens einen Einweg-Behälter in den dritten Behälter entlang der Flussrichtung zu pumpen.

Das Bereitstellen eines Druck erzeugenden Mittels erlaubt dem Verwender, zumindest einen Teil des zumindest einen Mediums in einen anderen Bereich des Systems und/oder der Vorrichtung, beispielsweise in einen weiteren Behälter, über eine Hauptleitung zu befördern. Insbesondere kann das Druck erzeugende Mittel einen Materialfluss bzw. einen Strom eines Teils des zumindest einen Mediums und/oder ein Druckgefälle erzeugen.

Ein Druck erzeugendes Mittel kann insbesondere zumindest eine Pumpe sein. Die zumindest eine Pumpe kann insbesondere eine Schlauchpumpe, Membranpumpe, Zentrifugalpumpe und/oder eine Peristaltikpumpe umfassen. Jede andere bekannte Pumpe zur Erzeugung eines Druckgefälles und/oder eines Materialflusses bzw. -stroms ist außerdem denkbar. Bevorzugt umfasst ein Druck erzeugendes Mittel zumindest eines aus: ein single-use bzw. Einweg-Sicherheitsventil, optional mit Feder, ein single-use check valve bzw. Einweg-Rückschlagventil bzw. Rückschlagventil zur Einwegnutzung, eine Berstscheibe mit "internem Loop" bzw. zumindest teilweise integrierter Bypass- und/oder Ableitung, eine Berstscheibe mit "externem Loop" bzw. nicht integrierter externer Bypass- und/oder Ableitung.

Ein ähnliches Druck erzeugendes Mittel kann auch eine mechanische Presse bzw. Kolben und/oder ein anderes Mittel sein, welche einen flexiblen Einweg-Behälter zusammendrücken, beispielsweise durch mechanischen Anpressdruck mittels Betätigung und/oder mittels Gewichtskraft.

Ein Druck erzeugendes Mittel kann auch ein Gefälle bzw. eine Anordnung mit Gefälle und/oder ein bezüglich der Gravitationsrichtung Gefälle-erzeugender Träger bzw. erzeugende technische Ausrüstung und/oder Einrichtung umfassen. Beispielsweise kann der mit einem Medium zumindest teilweise gefüllte Einweg-Behälter fluidisch mit einem anderen Behälter verbunden und gegenüber diesem etwas erhöht positioniert sein, sodass die Gewichtskraft des Mediums selbst dafür sorgt, dass ein Druck besteht, der einen Materialfluss bzw. einen Strom zumindest eines Teils des Mediums von dem Einweg-Behälter zu dem anderen Behälter erzeugt.

Ein Druck erzeugendes Mittel kann auch eine Wärmequelle umfassen, welche durch Einwirkung auf zumindest einen Teil des zumindest einen Mediums für dessen Ausdehnung oder gar für dessen Änderung des Aggregatszustandes, beispielsweise von einer Flüssigkeit zu einem Gas, sorgt. Die Ausdehnung dessen erzeugt, sofern der Behälter sein Volumen im Wesentlichen beibehält, einen Druck, der ebenfalls in der Lage sein kann, einen Materialfluss bzw. einen Strom zumindest eines Teils des Mediums von dem Einweg-Behälter zu dem anderen Behälter zu erzeugen.

Gemäß einem Aspekt ist das mindestens eine Druck erzeugende Mittel, insbesondere eine Pumpe, an der Hauptleitung angeordnet und ausgelegt, ein Druckgefälle entlang der Hauptleitung zu erzeugen.

Das Bereitstellen einer Pumpe an der Hauptleitung hat den Vorteil, dass beispielsweise ein Teil des zumindest einen Mediums aus einem bezüglich der Stromrichtung der Pumpe vorgelagerten Abschnitt des Systems in die Hauptleitung hineingesogen werden kann. Der Teil des zumindest einen Mediums, welcher die Pumpe passiert, kann im Anschluss in einen der Pumpe nachgelagerten Abschnitt des Systems beispielsweise durch einen Filter in einen weiteren Behälter befördert und/oder gepumpt werden.

Gemäß einem Aspekt ist das mindestens eine Druck erzeugende Mittel an dem Einweg-Behälter angeordnet und ausgelegt, ein Druckgefälle innerhalb des Einweg-Behälters und/oder entlang der Hauptleitung zu erzeugen.

Die genannte Ausführungsform hat den Vorteil, dass ein Einweg-Behälter druckbeaufschlagt befüllt und deshalb der Vorgang des Befüllens beschleunigt werden kann.

Gemäß einem Aspekt ist bzw. sind die Ableitung und/oder die Bypass-Leitung und insbesondere die Überdrucksicherung bezüglich der Flussrichtung hinter dem Druck erzeugenden Mittel und/oder an einer Hauptleitung angeordnet. Alternativ oder zusätzlich kann die mindestens eine Überdrucksicherung an einem anderen Bauteil der Vorrichtung und/oder des Systems als dem Einweg-Behälter und/oder der Hauptleitung angeordnet sein, beispielsweise an einem dritten Behälter.

Die genannte Anordnung der Ableitung und/oder der Bypass-Leitung unmittelbar nach bzw. hinter dem Druck erzeugenden Mittel bezüglich einer Strom- bzw. Flussrichtung hat den Vorteil, dass zumindest ein Teil des Mediums ab- und/oder zurückgeführt werden kann, wenn die Überdrucksicherung ausgelöst wird. Dies kann beispielsweise durch eine zu hohe Pumpleistung oder durch eine Verstopfung in einem Filter und/oder in der Hauptleitung ausgelöst werden. Insbesondere ist eine Überdrucksicherung an der Ableitung und/oder der Bypass-Leitung angeordnet.

Gemäß einem Aspekt umfasst das System ferner eine Rückkopplung, insbesondere eine elektrische Rückkopplung, bevorzugt zwischen Überdrucksicherung und Druck erzeugendem Mittel, wobei die Rückkopplung ausgelegt ist, bei der Auslösung der mindestens einen Überdrucksicherung, das Druck erzeugende Mittel abzuschalten. In anderen Worten verfügt die besagte Überdrucksicherung vorzugsweise über eine Rückkopplung, welche ein Signal, bevorzugt ein elektrisches Signal, nach dem Auslösen an die Pumpe senden kann, wobei das Signal die Pumpe veranlasst abzuschalten und die Pumpleistung einzustellen. Diese Ausführungsform hat den Vorteil ein, ein besonders sicheres System bereitzustellen.

Gemäß einem Aspekt ist bzw. sind die Ableitung und/oder die Bypass-Leitung und insbesondere die Überdrucksicherung zumindest teilweise in dem Druck erzeugenden Mittel integriert.

Die zuvor genannte Ausführungsform hat den Vorteil, dass Bauteile ineinander verarbeitet bzw. integriert sein können, was die Verwendung besonders einfach gestalten kann. Ferner kann derart die nachträgliche Montage durch den Verwender vermieden werden, sodass eine besonders komfortable Verwendung erlaubt wird.

Gemäß einem Aspekt ist bzw. sind mindestens der Einweg-Behälter und/oder die Überdrucksicherung sterilisierbar, insbesondere zumindest teilweise aus einem Kunststoff und/oder einem Edelstahl ausgebildet. In anderen Worten ist bzw. sind der Einweg-Behälter und/oder die Überdrucksicherung und/oder die gesamte Vorrichtung und/oder das System zumindest teilweise ausgelegt, unter Einsatz eines validierten Verfahrens sterilisiert werden zu können, vorzugsweise durch mindestens eines aus Gammabestrahlung, Autoklavieren, Dampfsterilisation und chemische Sterilisationsmittel.

Ein sterilisierbares Element hat den Vorteil, dass es in Kontakt mit Medien, beispielsweise Zellkulturen, treten kann, welche beispielsweise nicht durch biologische Substanzen kontaminiert werden sollen. Es ist darüber hinaus von Vorteil, wenn Bauteile bzw. Elemente einer Vorrichtung, insbesondere Einweg-Bauteile, bereits ab Verkauf bzw. ab Werk bereits sterilisiert vorliegen und sterilisiert verkauft werden, sodass der Verwender keine Maßnahmen zur Sterilisation ergreifen muss, bevor er die Vorrichtung und/oder das System in Betrieb nimmt.

Gemäß einem Aspekt umfasst die Überdrucksicherung ferner ein Ventil zum Ausgleich von Druckverhältnissen bzw. zum Ablassen bzw. Reduzieren eines Drucks durch Materialfluss in eine vorbestimmte bzw. vorbestimmbare Richtung. Ferner kann die Überdrucksicherung eine Rückflussverhinderungsvorrichtung zum Verhindern eines Rückflusses eines bereits durch eine Überdrucksicherung ausgetretenen Mediums umfassen.

Gemäß einem Aspekt weist die Überdrucksicherung einen Auslösedruck auf, welcher einem Wert des Drucks entspricht, den die Überdrucksicherung maximal toleriert bzw. aushält bzw. dem die Überdrucksicherung höchstens standhält und ab dessen Überschreiten die Überdrucksicherung ausgelöst wird. Der Auslösedruck ist vorzugsweise so eingestellt und/oder gewählt, dass er im Wesentlichen niedriger als der maximal zulässige Druck bzw. der Berstdruck des Behälters und/oder der Vorrichtung und/oder des Systems ist, insbesondere an der Stelle, an der die Überdrucksicherung an der Vorrichtung angeordnet ist.

Sofern der Auslösedruck einen Wert aufweist, der geringer ist als der maximal zulässige Druck bzw. der Berstdruck des Behälters, so kann im Normalfall ein Zerbersten und/oder Brechen und/oder Reißen und/oder eine Beschädigung des Behälters und/oder der Vorrichtung und/oder des Systems verhindert werden. Damit kann auch verhindert werden, dass ein Teil des Mediums unkontrolliert aus dem Behälter und/oder der Vorrichtung und/oder dem System austritt und stattdessen kontrolliert abgeführt werden kann.

Gemäß einem Aspekt umfasst die Überdrucksicherung mindestens eine Schicht und/oder ein Material und/oder eine Film- bzw. Lagenschicht, bevorzugt mit Sollbruchstellen, wobei die Schicht und/oder das Material und/oder die Film- bzw. Lagenschicht so konfiguriert ist bzw. sind, dass selbige bricht bzw. brechen, wenn sie einem Druck ausgesetzt wird bzw. werden, der den Auslösedruck übersteigt.

Gemäß einem Aspekt besteht die Überdrucksicherung in einem Abschnitt der Behälterwandung des Einweg-Behälters, wobei die Behälterwandung zumindest teilweise eine Folie umfasst und die Überdrucksicherung Teil dieser Folie ist und eine Folienstruktur der Folienschicht aufweist, die in mindestens einer Position geschwächt ist bzw. eine Sollbruchstelle aufweist, um den Auslösedruck einzustellen bzw. um ausgelöst zu werden wenn der Auslösedruck überschritten wird. Bevorzugt werden die Folienstruktur und insbesondere die Sollbruchstelle durch Dehnung, Perforation, chemische Verarbeitung und/oder physikalische Verarbeitung erzeugt. Insbesondere ist die Überdrucksicherung gemäß dieser Ausführungsform einstückig mit dem Einweg-Behälter ausgeformt.

Es besteht ein Vorteil darin, die Überdrucksicherung als Bestandteil der Folie auszubilden, da derart Material und/oder Aufwand und/oder Kosten eingespart werden kann. Die zuvor genannte Ausführungsform ermöglicht eine besonders simple bzw. einfache und/oder kostengünstige Lösung mit erhöhter Sicherheit.

Gemäß einem Aspekt ist die mindestens eine Überdrucksicherung, insbesondere die Berstscheibe und/oder die Folienschicht mit Sollbruchstelle ausgelegt fragmentfrei zu brechen, wenn sie ausgelöst wird bzw. werden.

Ein fragmentfreies Zerbersten einer Überdrucksicherung kann verhindern, dass Splitter und/oder Fragmente und/oder kleine Partikel und Bestandteile der Überdrucksicherung in das Medium und/oder die Vorrichtung und/oder das System und/oder die Pumpe gelangen. Somit kann vermieden und/oder verhindert werden, dass diese nach dem Zerbersten aufbereitet und/oder gereinigt und/oder von den Fragmenten befreit werden müssen, was mit hohem Aufwand verbunden sein kann.

Gemäß einem Aspekt umfasst die Vorrichtung ferner mindestens einen Sensor, der ausgelegt ist, anzuzeigen, wenn die Überdrucksicherung ausgelöst wurde und insbesondere die Sollbruchstelle gebrochen ist, nachdem der Auslösedruck überschritten wurde.

Ein Sensor, der anzeigen kann, ob die Überdrucksicherung ausgelöst wurde, kann beispielsweise auch dazu ausgelegt sein, ein Signal an ein anderes Element der Vorrichtung und/oder des Systems senden, um ein Element beispielsweise eine Pumpe und/oder einen Vorgang abzustellen. Deshalb weist diese besondere Ausführungsform eine erhöhte Sicherheit für den Verwender auf.

Gemäß einem Aspekt umfasst die Überdrucksicherung, insbesondere die Berstscheibe und/oder die Folienschicht mit Sollbruchstelle, mindestens eine Membran und/oder einen Membranfilter, wobei die Membran und/oder der Membranfilter ausgelegt ist bzw. sind, einen Auslösedruck aufzuweisen, der im Wesentlichen dem maximal zulässigen bzw. dem Berstdruck entspricht. Somit kann beispielsweise nur ein Bestandteil des zumindest einen Mediums durch die Überdrucksicherung austreten, während andere Bestandteile des Mediums im System und/oder der Vorrichtung verbleiben. Beispielsweise kann ein Medium einen Feststoff und eine Flüssigkeit umfassen, beispielsweise Festkörper-Schwebeteilchen, die in einer Flüssigkeit vorliegen. Falls der Auslösedruck der Membran und/oder des Membranfilters überschritten wird, kann die Flüssigkeit austreten und abgeführt werden während der Feststoff bzw. die Festkörper in dem System und/oder der Vorrichtung verbleiben. Dies ist insbesondere dann von Vorteil, wenn der Stoff, der in dem System und/oder der Vorrichtung verbleiben soll besonders empfindlich beispielsweise gegenüber Schwankungen (z.B. Temperatur, Druck, Atmosphäre, etc.) ist.

Gemäß einem Aspekt kann eine Berstscheibe mittels einer Tri-Clamp-Verbindung in oder an dem Einweg-Behälter und/oder der Vorrichtung und/oder dem System eingebaut und/oder angeordnet und/oder angebracht sein. Gemäß einem weiteren Aspekt können zumindest eine Ableitung und/oder zumindest eine Bypass-Leitung und/oder zumindest eine Hauptleitung eine "Kunststoff Pipe" Vorrichtungen umfassen und/oder darstellen.

Tri-Clamp-Verbindungen sind universell verwendbar und können als im Wesentlichen kostengünstige Verbindungen bereitgestellt werden.

Die Erfindung betrifft ferner ein Verfahren zum zumindest teilweisen Auffangen mindestens eines Mediums in einer Vorrichtung umfassend die Schritte:
- Bereitstellen mindestens eines Einweg-Behälters, der ausgelegt ist, das mindestens eine Medium zumindest teilweise aufzunehmen;
- Bereitstellen mindestens eine Überdrucksicherung, welche fluidisch mit dem mindestens einem Einweg-Behälter verbunden ist; und
für den Fall, dass die Überdrucksicherung ausgelöst wird:
- Ableiten des mindestens einen Mediums zumindest teilweise in einen weiteren zweiten Behälter mittels einer Ableitung; und/oder
- Rückführen des mindestens einen Mediums zumindest teilweise in einen bezüglich einer Stromrichtung der Überdrucksicherung vorgelagerten Vorrichtungsbereich, insbesondere in den mindestens einen Einweg-Behälter, mittels einer Bypass-Leitung.

Besondere Ausführungsformen des Verfahrens können ferner Merkmale der zuvor genannten Ausführungsformen einer Vorrichtung und/oder eines Systems aufweisen. Ein Verfahren kann entsprechend die zuvor genannten Vorteile der einzelnen Merkmale haben.

Die Erfindung wird nachfolgend anhand von in Figuren gezeigten Ausführungsbeispielen näher erläutert. Einzelne, in den Figuren gezeigte Merkmale können mit anderen Ausführungsbeispielen kombiniert werden, sofern sie sich nicht gegenseitig ausschließen. Gleiche Bezugszeichen bezeichnen dabei gleiche oder ähnliche Bauteile der Ausführungsformen. Es zeigen:
**Fig. 1** eine schematische Seitenansicht eines Querschnitts einer Vorrichtung umfassend einen Einweg-Behälter, eine Überdrucksicherung und eine Ableitung gemäß einer Ausführungsform;
**Fig. 2** eine schematische Seitenansicht eines Querschnitts einer Vorrichtung umfassend einen Einweg-Behälter, eine Überdrucksicherung und eine Ableitung gemäß einer weiteren Ausführungsform;
**Fig. 3** eine schematische Seitenansicht eines Systems mit einer Vorrichtung umfassend einen Einweg-Behälter, eine Pumpe, eine Überdrucksicherung und eine Bypass-Leitung gemäß einer Ausführungsform;
**Fig. 4** eine schematische Seitenansicht eines Systems mit einer Vorrichtung umfassend einen Einweg-Behälter, eine Pumpe, eine Überdrucksicherung und eine Bypass-Leitung gemäß einer weiteren Ausführungsform;
**Fig. 5** eine schematische Seitenansicht eines Systems mit einer Vorrichtung umfassend einen Einweg-Behälter, ein Druck erzeugendes Mittel, eine Überdrucksicherung und eine Bypass-Leitung gemäß einer weiteren Ausführungsform;
**Fig. 6** eine schematische Seitenansicht eines Systems mit einer Vorrichtung umfassend einen Einweg-Behälter, eine Pumpe, eine Überdrucksicherung und eine Ableitung gemäß einer Ausführungsform;
**Fig. 7** eine schematische Seitenansicht eines Systems mit einer Vorrichtung umfassend einen Einweg-Behälter, eine Pumpe, eine in die Pumpe integrierte Überdrucksicherung und eine in die Pumpe integrierte Bypass-Leitung gemäß einer Ausführungsform;
**Fig. 8** eine schematische Seitenansicht eines Systems mit einer Vorrichtung umfassend einen Einweg-Behälter, eine Pumpe, eine in die Pumpe integrierte Überdrucksicherung und eine Bypass-Leitung gemäß einer Ausführungsform;
**Fig. 9** eine Berstscheibe in einem offenen Zustand nach deren Auslösung und eine Berstscheibe in einem geschlossenen Zustand vor deren Auslösung gemäß einer Ausführungsform;
**Fig. 10** ein Rückschlagventil gemäß einer Ausführungsform.

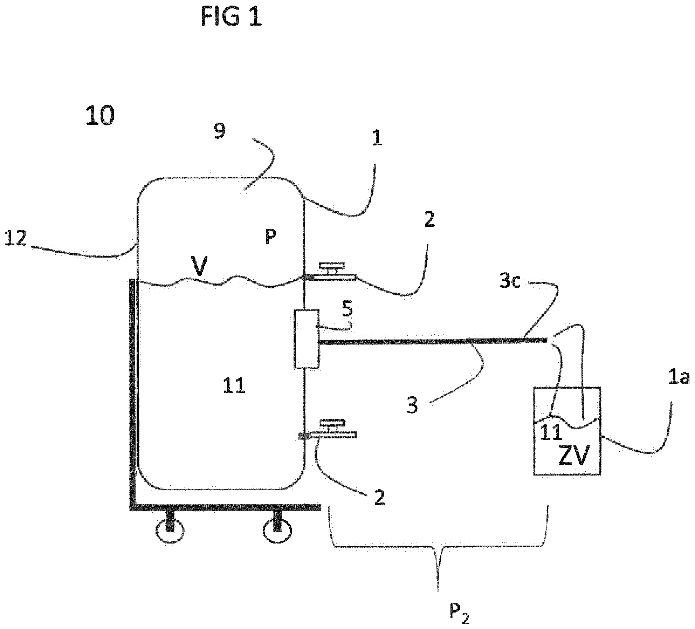

Die **Fig. 1** zeigt eine Vorrichtung 10 gemäß einer Ausführungsform umfassend zumindest einen Einweg-Behälter 1 mit einem Volumen V und zumindest einer Überdrucksicherung 5. Die Überdrucksicherung 5 ist an der Behälterwandung 12 des Einweg-Behälters 1 angeordnet und insbesondere darin integriert. Ferner umfasst der Einweg-Behälter 1 zwei Zu- und/oder Abläufe 2, durch die ein Medium in den Einweg-Behälter 1 eingeführt und/oder abgeführt werden kann. In einer hier nicht gezeigten Ausführungsform umfasst ein Einweg-Behälter 1 mehr als zwei Zu- und/oder Abläufe 2 oder nur einen Zu- und/oder Ablauf. In anderen Worten kann der Einweg-Behälter 1 über die Zu- und/oder Abläufe 2 mit dem Medium befüllt und/oder von diesen (zumindest teilweise) entleert werden. Hierbei kann das Medium ein Fluid (insbesondere eine Flüssigkeit und/oder Gas) und/oder einen Feststoff sein bzw. umfassen.

In dem Einweg-Behälter 1 befindet sich insbesondere zumindest ein flüssiges Medium 11 und ein darüber stehendes Gas. In dem Einweg-Behälter 1 herrscht ein mittlerer Druck P, der Partialdrücke umfassen kann, die sich jedoch bevorzugt im Wesentlichen nicht stark (z.B. um nicht mehr als etwa 10%, bevorzugt nicht mehr als etwa 1%) voneinander unterscheiden. Der Einweg-Behälter 1 bildet mit der Überdrucksicherung 5 ein im Wesentlichen geschlossenes bzw. hermetisch abgeschlossenes bzw. von einer Umgebung getrenntes System 9, sofern die Überdrucksicherung 5 nicht ausgelöst wurde. Die Überdrucksicherung 5 kann insbesondere eine Berstscheibe und/oder eine Membran und/oder einen Membranfilter umfassen. Alternativ oder zusätzlich kann die Überdrucksicherung 5 einen Abschnitt der Behälterwandung 12 darstellen, welcher eine Strukturierung, insbesondere Sollbruchstelle aufweist.

Sobald der Druck P in dem geschlossenen System einen spezifischen (vorbestimmten bzw. vorbestimmbaren) Auslösedruck der Überdrucksicherung 5 übersteigt, öffnet und/oder zerberstet bzw. bricht die Überdrucksicherung 5, sodass aus dem geschlossenen System ein offenes System, insbesondere mit einer Verbindung bezüglich des Drucks zur Außenseite, wird. Zumindest ein Teil des zumindest einen Mediums kann durch die Überdrucksicherung 5 treten und entlang einer Leitung 3, welche hier einer Ableitung 3c entspricht und welche ein Rohr umfasst, in zumindest ein Zielvolumen ZV eines weiteren zweiten Behälters 1a, insbesondere eines Auffangbehälters gelangen. In der gezeigten Ausführungsform ist der Weg, den zumindest ein Teil des Mediums 11 durch die Ableitung 3c zu dem Auffangbehälter 1a nimmt, kein geschlossenes System. Nachdem der Pegel des Mediums innerhalb des Einweg-Behälters 1 unterhalb der Höhe des unteren der beiden Zu- und/oder Abläufe 2 gelangt, kann auch ein Gas entweichen.

Ein Einweg-Behälter 1 ist ein Behälter, der für eine einmalige Verwendung konfiguriert ist. Nachdem der Einweg-Behälter 1 einmal benutzt wurde, hat er üblicherweise seine Funktion erfüllt und kann entsorgt werden. Beispielhaft ist ein Einweg-Behälter 1 aus Kunststoff hergestellt, der Polyamid, Polycarbonat, Polyethylen, Polystyrol, Polyethersulfon, Polypropylen, Polytetrafluorethylen, Polyvinylchlorid, Celluloseacetat und/oder Ethylvinylacetat umfassen kann, aber nicht darauf beschränkt ist. In einem Beispiel kann der Einweg-Behälter 1 im Wesentlichen starr sein, d. H. seine Form kann nicht modifiziert werden. In einem anderen Beispiel kann der Einweg-Behälter 1 (zumindest teilweise) flexible Wände bzw. eine flexible Behälterwandung 12 aufweisen, d. H. der Einweg-Behälter 1 kann seine Form ändern, ohne zu brechen.

Einweg-Behälter 1 können beispielhaft für kritische Anwendungen insbesondere in der biopharmazeutischen und bioproduzierenden Industrie verwendet werden. Die Verwendung eines der Einweg-Behälter 1 kann Folgendes einschließen, ohne jedoch darauf beschränkt zu sein: Lagerung eines Mediums (z.B. Produkts), Mischen und/oder Zellkultivierung. Der der Einweg-Behälter 1 ist insbesondere ein sterilisierbarer der Einweg-Behälter 1 aus Kunststoff, der ausgelegt ist, mindestens eine Flüssigkeit aufzunehmen oder zu halten. Es kann ein Bioreaktorbeutel, ein Mischbehälter, ein 2D- und/oder ein 3D-Bioprozessbeutel sein bzw. umfassen.

Beispielhaft kann ein Einweg-Behälter 1 ein Gehäuse bzw. eine Behälterwandung 12 mit einer Mehrschichtfolienstruktur umfassen, d. H. eine Überlagerung von dünnen Schichten aus Kunststoffmaterialien, die eine sichere Barriere zwischen dem Inhalt des Einweg-Behälters 1 (z. B. biogefährdendes Material) und der äußeren Umgebung bereitstellt. Ferner kann ein Einweg-Behälter 1 beispielhaft vorsterilisiert bereitgestellt werden (z. B. mittels Gammabestrahlung und/oder Autoklavieren). Ein Einweg-Behälter 1 kann somit eine vorteilhafte Alternative zu herkömmlichen Glas- und/oder Edelstahlsystemen darstellen.

Der Einweg-Behälter 1 kann zumindest teilweise zumindest ein Medium 11 umfassen, welches ein Fluid enthält, beispielsweise ein Gas, eine Flüssigkeit und/oder eine Mischung daraus, wobei die Phasen getrennt und/oder im Wesentlichen "gemischt" vorliegen können. Der Druck P in dem Einweg-Behälter 1 wirkt im Wesentlichen auf die Wände des Gehäuses bzw. die Behälterwandung 12 des Einweg-Behälters 1. In einem Beispiel kann das Gehäuse 100 ein Biostat^{®} Cultibag^{®} STR-Bioreaktorbeutel oder ein Flexsafe/Flexel mixing/storage flexboy etc. sein.

Die Überdrucksicherung 5 ist strömungsmittelmäßig bzw. fluidisch mit dem Einweg-Behälter 1 und insbesondere dessen Behälterwandung 12 verbunden, indem ein Fluidfluss bzw. ein Materialfluss von der Behälterwandung 12 zu der Überdrucksicherung 5 ermöglicht wird. In anderen Worten gibt es im Wesentlichen kein unter normalen Umständen unüberwindbares Hindernis, welches das Medium daran hindert von der Behälterwandung 12 zu der Überdrucksicherung 5 zu fließen. In einem Beispiel kann die Überdrucksicherung 5 unmittelbar in die Behälterwandung 12 integriert sein, z. B. kann die Behälterwandung 12 integral an der Behälterwandung 12 ohne mechanisches Verbindungsstück angebracht und/oder angeordnet sein.

Die Überdrucksicherung 5 kann z.B. zumindest teilweise innerhalb einer Mehrschichtstruktur der Behälterwandung 12 positioniert und/oder angeordnet sein (z. B. kann ein Flansch in eine oder mehrere Schichten der Mehrschichtstruktur eingebettet und/oder verlötet sein).

In einem anderen Beispiel kann die Überdrucksicherung 5 "extern", insbesondere mittels eines Verbindungsstücks mit der Behälterwandung 12 verbunden sein. Die Überdrucksicherung 5 kann z.B. an einem externen weiteren Behälter 1b und/oder an einer Leitung angeordnet sein, welche jeweils mit der Behälterwandung 12 mittels einer Verbindung, wie etwa einer Schlauchverbindung, verbunden sein können.

Verbindungsstücke und/oder Verbindungen, wie Leitungen können bevorzugt aseptische Verbindungen umfassen, wie beispielsweise aseptische Konnektoren, insbesondere OPTA ^{®} Konnektoren. In einem Beispiel ist die Überdrucksicherung 5 ausgelegt, vor deren Montage an dem Einweg-Behälter 1 sterilisiert zu werden, z. B. mittels Gammabestrahlung, chemisches Sterilisationsmittel (wie mit verdampftem Wasserstoffperoxid, Ethylenoxid usw.), Dampfsterilisation und/oder Autoklavieren. In einem anderen Beispiel kann die Überdrucksicherung 5 zusammen mit Einweg-Behälter 1, insbesondere dessen Behälterwandung 12 sterilisiert werden.

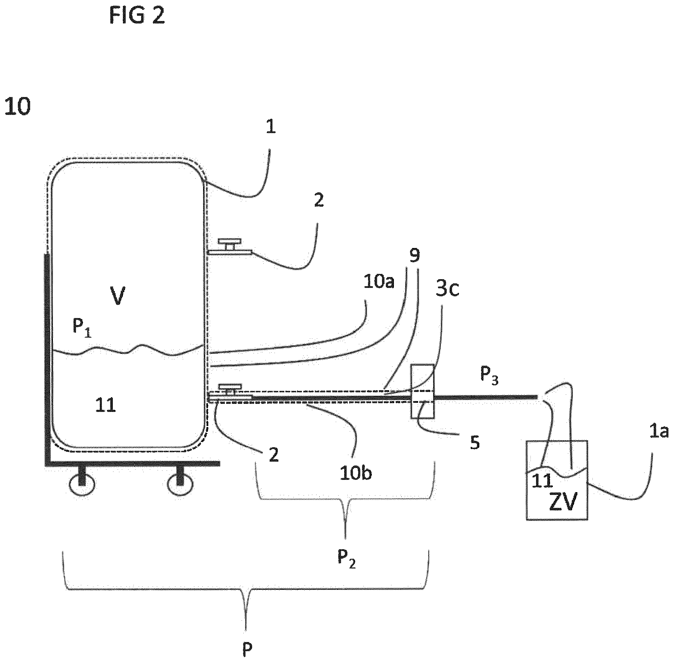

Die **Fig. 2** zeigt eine Vorrichtung 10 gemäß einer Ausführungsform umfassend einen Einweg-Behälter 1 mit einem Volumen V und einer Leitung, welche einer Ableitung 3c entspricht, an der eine Überdrucksicherung 5 angeordnet ist. Ferner umfasst der Einweg-Behälter 1 insbesondere zwei Zu- und/oder Abläufe 2, wobei die Leitung an einem der beiden Zu- und/oder Abläufe 2 angeordnet bzw. anordenbar ist. In dem Einweg-Behälter 1 befindet sich insbesondere zumindest ein flüssiges Medium 11 und ein darüber stehendes Gas, welches als ein weiteres Medium betrachtet werden kann. Der Einweg-Behälter 1 kann als ein erstes Teilsystem 10a betrachtet werden, welches offen oder geschlossen sein kann, je nachdem, ob einer oder beide der Zu- und/oder Abläufe 2 jeweils offen oder geschlossen ist bzw. sind. Die Leitung kann als ein zweites Teilsystem 10b betrachtet werden, welches offen oder geschlossen sein kann, je nachdem, ob der Zu- und/oder Ablauf 2 an dem die Leitung angeordnet ist, jeweils offen oder geschlossen ist.

Sofern der Zu- und/oder Ablauf 2, an dem die Leitung angeordnet ist, geöffnet ist, bildet die Leitung mit dem Einweg-Behälter 1 ein geschlossenes System, in dem ein mittlerer Gesamtdruck P vorherrscht und welches durch eine gestrichelte Linie angedeutet wird. Es kann in dem ersten offenen oder geschlossenen System 10a umfassend den Einweg-Behälter 1 ein Druck, insbesondere ein Partialdruck P₁ vorherrschen. Es kann in dem zweiten offenen oder geschlossenen System 10b umfassend die Leitung ein Druck, insbesondere ein Partialdruck P₂ vorherrschen. Im Normalfall kann ein fluides Medium 11 beim Einfüllen, beispielsweise durch den oberen Zu- und/oder Ablauf 2 in die Leitung fließen, wo es von der Überdrucksicherung 5 daran gehindert wird, der Leitung zu entweichen, sofern der Druck P in der Vorrichtung, insbesondere der Druck P₁ in der Leitung nicht den Auslösedruck übersteigt.

In dem Einweg-Behälter 1 herrscht ein mittlerer Druck P, der Partialdrücke P₁ und P₂ umfassen kann, die sich, sofern die Systeme miteinander in Verbindung stehen, jedoch bevorzugt im Wesentlichen nicht stark (z.B. um nicht mehr als etwa 10%, bevorzugt nicht mehr als etwa 1%) voneinander unterscheiden. Der Einweg-Behälter 1 bildet mit der Überdrucksicherung 5 ein im Wesentlichen geschlossenes bzw. hermetisch abgeschlossenes System 9, sofern die Überdrucksicherung 5 nicht ausgelöst wurde.

Sobald der Druck P, insbesondere der Partialdruck P₂ in dem geschlossenen System den speziellen Auslösedruck der Überdrucksicherung 5 übersteigt, öffnet und/oder zerberstet bzw. bricht die Überdrucksicherung 5, sodass aus dem geschlossenen System aus den beiden Teilsystemen 10a und 10b ein offenes System, insbesondere mit einer Verbindung bezüglich des Drucks zur Außenseite, wird. Zumindest ein Teil des zumindest einen Mediums kann durch die Überdrucksicherung 5 treten und entlang der Leitung, in ein Zielvolumen ZV eines zweiten Behälters 1a gelangen. Auch in dieser gezeigten Ausführungsform ist der Weg, den zumindest ein Teil des Mediums 11 durch die Ableitung 3c zu dem Auffangbehälter 1a nimmt, kein geschlossenes System.

Das austretende Medium 11 kann derart kontrolliert, definiert und/oder geführt durch die Leitung in einen Auffangbehälter 1a geleitet werden. Somit kann vermieden werden, dass das austretende Medium 11 verloren geht. Ferner kann vermieden werden, dass das Medium in die Umgebung gelangt und diese gegebenenfalls kontaminiert.

Hinter der Überdrucksicherung 5 herrscht ein Druck P₃, welcher, sofern das System ein offenes darstellt, ein lokaler Druck der Umgebung sein kann. Alternativ kann das System auch hinter der Überdrucksicherung 5 ein geschlossenes System mit einem kontrollierten und/oder vorbestimmten Druck darstellen. Dies ist insbesondere dann von Vorteil, wenn das austretende Medium 11 Substanzen enthält, die flüchtig sind, also leicht von einem flüssigen in einen gasförmigen Zustand übergehen und/oder Medien und/oder Substanzen, die nicht mit der Umgebung in Berührung kommen sollen. Dasselbe gilt für ein Medium 11, welches ein Gas umfasst.

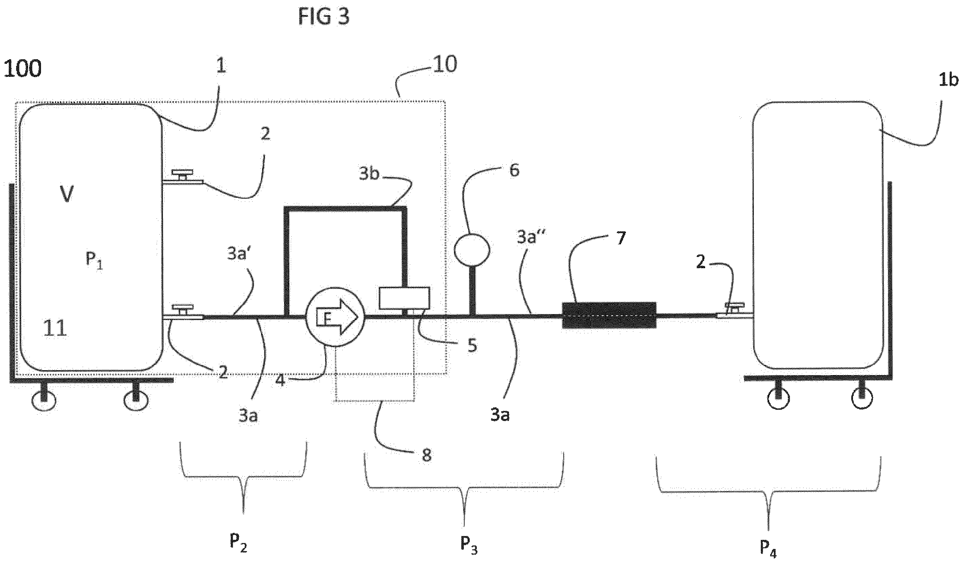

Die **Fig. 3** zeigt ein System 100 gemäß einer Ausführungsform, welches eine Vorrichtung 10 umfasst. Die Vorrichtung 10 umfasst einen Einweg-Behälter 1 mit einem Volumen V, welches dazu ausgelegt ist, zumindest ein Medium 11 zumindest teilweise aufzunehmen. Der Einweg-Behälter 1 verfügt über zwei Zu- und/oder Abläufe 2, die gesondert jeweils per Hand und/oder automatisch (z.B. durch eine nicht gezeigte Steuer- bzw. Regeleinrichtung) geöffnet und/oder geschlossen werden können. In alternativen Ausführungsformen umfassen Einweg-Behälter mehr als zwei oder weniger als zwei Zu- und/oder Abläufe. An dem unteren Zu- und/oder Ablauf 2 ist eine Hauptleitung 3a angeordnet, welche dazu ausgelegt ist einen Materialfluss bzw. einen Strom umfassend zumindest einen Teil des zumindest einen Mediums 11 entlang einer lokalen Strom- bzw. Flussrichtung F zu führen.

Die Hauptleitung 3a verbindet im Wesentlichen den Einweg-Behälter 1 mit einem weiteren Behälter 1b, der auch als dritter Behälter 1b bezeichnet wird, wenn man annimmt, dass ein erster Behälter 1 dem Einweg-Behälter 1 und ein zweiter Behälter 1a einem Auffangbehälter entspricht. Das System 100 muss jedoch nicht notwendigerweise einen Auffangbehälter 1a umfassen. Zumindest ein Teil des zumindest einen Mediums 11 im Einweg-Behälter 1 kann entsprechend entlang der Hauptleitung und der Flussrichtung F durch ein Druck erzeugendes Mittel 4, insbesondere eine Pumpe, zu dem dritten Behälter 1b gelangen. Ferner passiert das zumindest eine Medium 11 zumindest teilweise einen Filter 7, welcher an der Hauptleitung 3a angeordnet ist und/oder einen Abschnitt der Hauptleitung 3a bildet. Durch einen Zu- und/oder Ablauf 2 des weiteren bzw. dritten Behälters 1b gelangt das zumindest eine Medium 11 zumindest teilweise in den Innenraum des weiteren bzw. dritten Behälters 1b. Bei dem weiteren bzw. dritten Behälter 1b kann es sich insbesondere auch um einen weiteren Einweg-Behälter handeln. Alternativ kann der weitere bzw. dritte Behälter 1b auch ein Mehrweg-Behälter sein oder einen solchen umfassen.

In dem Einweg-Behälter 1 herrscht im Wesentlichen ein durchschnittlicher Druck P₁, welcher insbesondere homogen bzw. räumlich konstant ist. In dem Abschnitt der Hauptleitung 3a zwischen dem Zu- und/oder Ablauf 2 des Einweg-Behälters 1 und der Pumpe 4 herrscht ein Druck P₂. Sofern der besagte Zu- und/oder Ablauf 2 des Einweg-Behälters 1 geöffnet ist, können sich die beiden Drücke P₁, P₂ im Wesentlichen gleichen oder nur gering verschieden sein.

Im Betrieb des Druck erzeugenden Mittels 4 wird insbesondere ein Druckgefälle entlang der Hauptleitung 3a erzeugt, sodass in dem Abschnitt der Hauptleitung 3a zwischen dem Zu- und/oder Ablauf 2 des Einweg-Behälters 1 und dem Druck erzeugenden Mittel 4 ein niedriger Druck P₂ herrscht als in dem Abschnitt "hinter" dem Druck erzeugenden Mittel 4 in Richtung des dritten Behälters 1b. Der Druck P₃ zwischen dem Druck erzeugenden Mittel 4 und dem Filter 7 ist entsprechend größer als der Druck P₂, der bezüglich der Flussrichtung F vor dem Druck erzeugenden Mittel 4 herrscht. Das Druckgefälle entlang der Hauptleitung 3a kann insbesondere im Wesentlichen kontinuierlich verlaufen oder sprungartig, insbesondere in unmittelbarer Nähe zum Druck erzeugenden Mittel 4 ansteigen. Bei im Wesentlichen kontinuierlichem Anstieg würde der gemittelte Druck P₃ beispielsweise verschiedene Partialdrücke umfassen, wohingegen bei einem sprunghaften Anstieg der Druck P₃ in dem Bereich zwischen Druck erzeugenden Mittel 4 und Filter 7 im Wesentlichen örtlich homogen verteilt ist.

Im Wesentlichen stellt ein Filter 7 für einen Materialfluss bzw. Strom einen Widerstand dar, weshalb bezüglich der Flussrichtung F "hinter" dem Filter 7 ein anderer Druck P₄ herrscht als davor. Es kann entsprechend sein dass P₄ einen kleineren Wert aufweist als P₃. Sofern im Wesentlichen keine Widerstande zwischen dem Filter 7 und dem dritten Behälter 1b vorliegen und der Zu- und/oder Ablauf 2 des dritten Behälters 1b geöffnet ist, kann der Druck P₄ im Wesentlichen örtlich im dritten Behälter 1b und in dem entsprechenden Abschnitt der Hauptleitung 3a homogen verteilt sein.

In der zuvor genannten Konfiguration fließt zumindest ein Teil des zumindest einen Mediums 11, angetrieben durch ein Druck erzeugendes Mittel 4 von dem Einweg-Behälter 1 in den dritten Behälter 1b. Der entsprechende Teil des zumindest einen Mediums 11 wird auf diesem Weg entlang der Flussrichtung F gefiltert, sodass möglicherweise Schwebeteilchen aus dem entsprechenden Teil des zumindest einen Mediums 11 gefiltert werden können. Darüber hinaus kann es sein, dass in dem Einweg-Behälter 1 mindestens ein chemischer und/oder biologischer und/oder biochemischer und/oder physikalischer Prozess stattfindet und ein Teil des zumindest einen Mediums, beispielsweise ein Bodensatz und/oder ein Überstand des Bodensatzes, welche sich während des mindestens einen Prozesses gebildet haben, in den weiteren Behälter 1b überführt werden soll bzw. sollen. In dem weiteren bzw. dritten Behälter 1b kann der überführte Teil des zumindest einen Mediums 11 von dem anderen in dem Einweg-Behälter 1 verbliebenen Teil des zumindest einen Mediums 11 räumlich getrennt werden. Ferner kann der in den dritten Behälter 1b überführte Teil des zumindest einen Mediums 11 weiterverarbeitet und/oder verworfen und/oder gelagert werden.

Der zuvor beschriebene Fall entspricht insbesondere einem normalen Betrieb des Systems 100. Es kann in seltenen Fällen zu Störungen kommen, insbesondere dann wenn der Druck an einer Stelle bzw. Position in dem System 100 einen Berstdruck des Systems 100 überschreitet. An der entsprechenden Stelle können infolgedessen Risse und/oder Brüche und/oder Leckagen entstehen, durch welche zumindest ein Teil des Mediums 11 austreten kann. An einer besonders fragilen und/oder kritischen Position, wo ein die Wahrscheinlichkeit gegeben ist, dass ein Druck einen Berstdruck des Systems 100 und/oder der Vorrichtung 10 überschreitet, kann zur Abhilfe eine Überdrucksicherung 5 eingebaut werden. In der **Fig. 3** ist eine solche kritische Stelle zwischen dem Druck erzeugenden Element 4 bzw. der Pumpe und dem Filter 7 zu finden. Es kann in einem Szenario eine zu hohe Pumpleistung an der Pumpe 4 eingestellt sein, sodass ein größeres Volumen des Mediums 11 gepumpt wird als es durch die Hauptleitung 3a abfließen kann. Dieser Zustand hat zur Folge, dass der Druck P₃ zwischen Pumpe 4 und Filter 7 stetig ansteigt. In einem weiteren Szenario kann es sein, dass der Filter verstopft ist und somit einen hohen Widerstand bildet, durch den der Druck P₃ vor dem Filter 7 ebenfalls stetig steigt. Auch in diesem Fall ist die Pumpleistung an der Pumpe 4, selbst wenn sie zu Beginn richtig eingestellt wurde, so hoch, sodass ein größeres Volumen des Mediums 11 gepumpt wird als es durch die Hauptleitung 3a und insbesondere den Filter 7 abfließen bzw. hindurchtreten kann. Sofern keine geeignete Überdrucksicherung 5 an der entsprechenden Stelle eingebaut ist, kann es dazu kommen, dass das System 100 an dieser Stelle bricht oder zerberstet. Entsprechend ist in dem System 100 der **Fig. 3** eine Überdrucksicherung 5 an der Hauptleitung 3a, insbesondere an einem Seitenarm der Hauptleitung 3a zwischen Pumpe 4 und Filter 7 angeordnet.

Sobald der Druck P₃ einen Auslösedruck der Überdrucksicherung 5 überschreitet, wird die Überdrucksicherung 5 ausgelöst und leitet zumindest einen Teil des zumindest einen Mediums 11 in eine Bypass-Leitung 3b ab. Die Bypass-Leitung 3b ist dabei ausgelegt, das entsprechende Medium 11 in einen - bezüglich der Fluss- bzw. Stromrichtung F in der Hauptleitung 3a - der Überdrucksicherung 5 vorgelagerten Abschnitt 3a' des Systems 100, und zwar der Hauptleitung 3a zurückzuführen. Dies ist möglich, wenn der Druck P₂ vor der Pumpe 4 einen niedrigeren Wert aufweist als der Druck P₃ zwischen Pumpe 4 und Filter 7. Auf diese Weise kann der Druck P₃ in einem - bezüglich der Stromrichtung F in der Hauptleitung 3a - der Überdrucksicherung 5 nachgelagerten Abschnitt 3a" des Systems 100, und zwar der Hauptleitung 3a vermindert oder zumindest beschränkt werden.

Besonders vorteilhaft ist eine Rückkopplung 8 zwischen der Überdrucksicherung 5 und der Pumpe 4, da die Rückkopplung 8 ein Signal sein kann, welches ausgelegt ist, die Pumpe 4 abzustellen und somit kein weiterer Druckaufbau mittels der Pumpe 4 zwischen Pumpe 4 und Filter 7 verursacht. Die Rückkopplung 8 kann beispielsweise ein Notschalter bzw. ein Notsignal sein, der in einem Notfall die Pumpe 4 und/oder das gesamte System 100, insbesondere alle darin ablaufenden Prozesse abschaltet. Die Rückkopplung 8 kann ferner auch ein anderes Signal sein und/oder umfassen, das einen Alarm auslösen kann.

Darüber hinaus kann ein Barometer 6, der an der Hauptleitung 3a zwischen der Pumpe 4 und dem Filter 7 angeordnet ist, einen lokalen Druck P₃ messen. Dieses Barometer 6 kann zur Verbesserung der Sicherheit des Systems 100 dienen, da ein Verwender im Betrieb des Systems 100 den Druck P₃ kontrollieren kann.

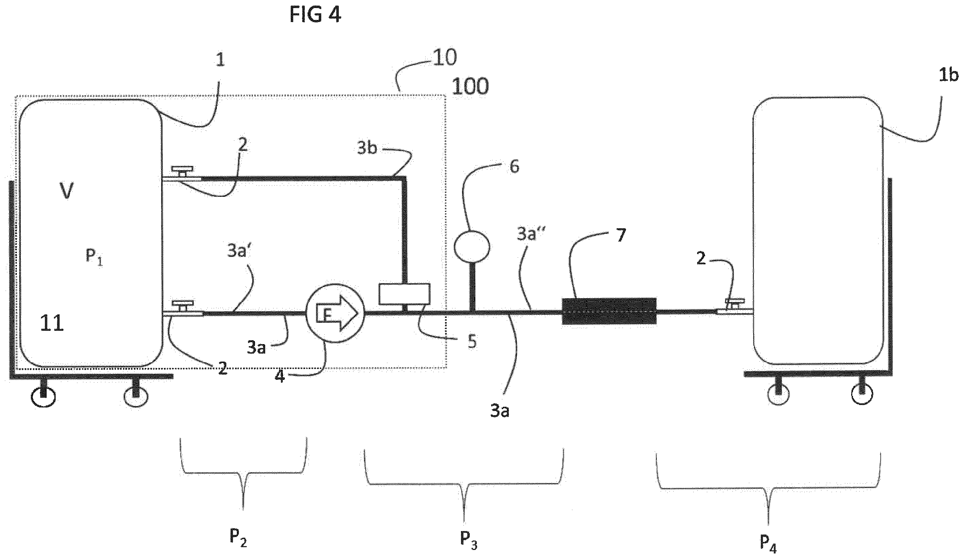

Die **Fig. 4** zeigt ebenfalls ein System 100 gemäß einer weiteren Ausführungsform, welches eine Vorrichtung 10 umfasst. In dieser gezeigten Ausführungsform wird, sofern der Druck P₃ einen Auslösedruck der Überdrucksicherung 5 überschreitet, die Überdrucksicherung 5 ausgelöst und zumindest ein Teil des zumindest einen Mediums 11 in die Bypass-Leitung 3b abgeführt. Die Bypass-Leitung 3b ist dabei ausgelegt, das entsprechende Medium 11 in einen - bezüglich der Fluss- bzw. Stromrichtung F in der Hauptleitung 3a - der Überdrucksicherung 5 vorgelagerten Abschnitt 3a' des Systems 100, und zwar den Einweg-Behälter 1 zurückzuführen. Dies ist dann möglich, wenn der Druck P₁ in dem Einweg-Behälter 1 einen niedrigeren Wert aufweist als der Druck P₃ zwischen Pumpe 4 und Filter 7. Derart kann der Druck P₃ in einem - bezüglich der Stromrichtung F in der Hauptleitung 3a - der Überdrucksicherung 5 nachgelagerten Abschnitt 3a" des Systems 100, und zwar der Hauptleitung 3a vermindert oder zumindest beschränkt werden.

Falls eine Rückkopplung, wie sie in Fig. 3 gezeigt ist, in dem System 100 integriert wäre, so könnte diese ein Signal an die Zu- und/oder Abläufe 2, insbesondere den unteren Zu- und/oder Ablauf 2 senden, um diese bzw. diesen zu verschließen, so dass kein weiteres Medium 11 mehr aus dem Einweg-Behälter 1 befördert werden kann. Eine solche Rückkopplung kann in dem System 100 integriert sein, was jedoch nicht zwingend der Fall sein muss. Die Rückkopplung ist in dieser Darstellung nicht gezeigt. Ferner könnte die Pumpe 4 optional mittels des Signals abgeschaltet werden. Darüber hinaus könnte auch ein Entlüftungsventil in dem System 100 mittels des Signals veranlasst werden, das System 100 zu belüften und zumindest einen Partialdruck des Drucks P im System 100 an den Außendruck anzugleichen, um ein weiteres möglicherweise unkontrolliertes Ansteigen des Drucks P im System 100 zu verhindern. Generell kann der Druck P einem mittleren Gesamtdruck und/oder einem der Partialdrücke P₁, P₂, P₃, P₄ entsprechen.

Durch das Umleiten bzw. Zurückleiten eines Teils des zumindest eine Mediums 11 in den Einweg-Behälter 1 kann dieser Teil vor einem unkontrollierten Austreten bewahrt werden. Insbesondere kann er dem Teil des Mediums 11, welcher in dem Einweg-Behälter 1 verblieben ist, wieder zugeführt werden.

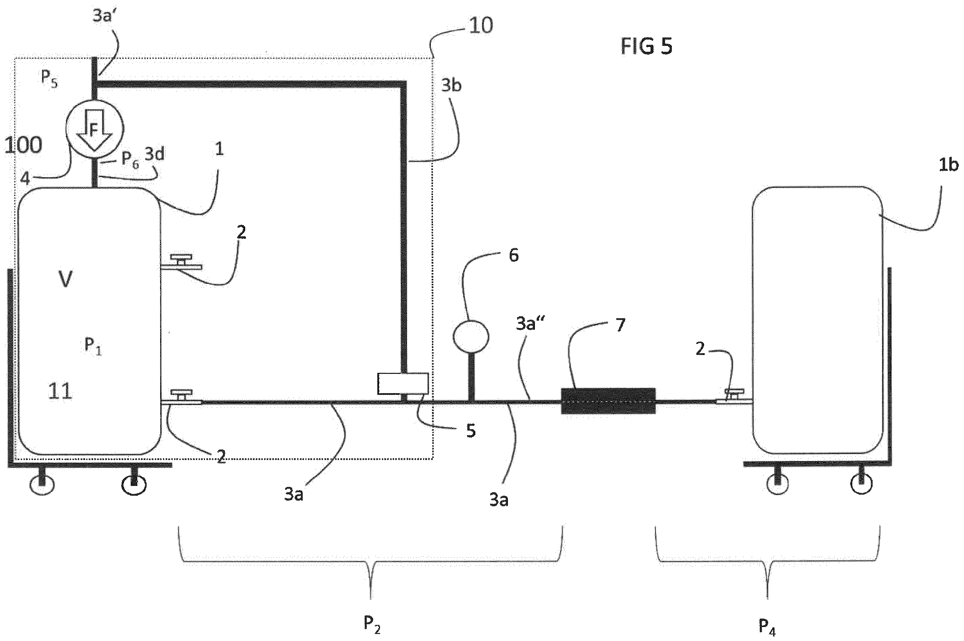

Die **Fig. 5** zeigt ebenfalls ein System 100 gemäß einer weiteren Ausführungsform, welches eine Vorrichtung 10 umfasst. In dieser gezeigten Ausführungsform ist zumindest ein Druck erzeugendes Mittel 4 an einer Stelle des Systems 100 angeordnet, an der ein Medium 11 in den Einweg-Behälter 1 gepumpt und/oder befördert und/oder gefüllt werden kann. Der Einweg-Behälter 1 kann entsprechend als "Druck beaufschlagter" Behälter erachtet werden. Das Druck erzeugende Mittel 4 kann auch in diesem Fall eine Pumpe umfassen und/oder darstellen. Es ist davon auszugehen, dass der Einweg-Behälter 1 über die Hauptleitung 3a fluidisch mit dem dritten Behälter bzw. dem weiteren Behälter 1b verbunden ist.

Das Druck erzeugende Mittel 4 befördert das zumindest eine in den Einweg-Behälter 1 zu füllende Medium 11 mit Druck P₆ in den Einweg-Behälter 1, sodass in dem Einweg-Behälter 1 ein Druck P₁ entsteht und/oder ansteigt.

Sofern der Druck P₂ in der Hauptleitung einen Auslösedruck der Überdrucksicherung 5 überschreitet, wird die Überdrucksicherung 5 ausgelöst und zumindest ein Teil des zumindest einen Mediums 11 in die Bypass-Leitung 3b abgeführt. Die Bypass-Leitung 3b ist dabei ausgelegt, das entsprechende Medium 11 in einen - bezüglich der Fluss- bzw. Stromrichtung F in der Hauptleitung 3a - der Überdrucksicherung 5 vorgelagerten Abschnitt 3a' des Systems 100, und zwar eine dem Einweg-Behälter 1 vorgelagerte Leitung zurückzuführen. Dies ist dann möglich, wenn der Druck P₅ in der dem Einweg-Behälter 1 vorgelagerten Leitung einen niedrigeren Wert aufweist als der Druck P₂ zwischen Einweg-Behälter 1 und Filter 7. Derart kann der Druck P₂ in der Hauptleitung 3a und/oder dem Einweg-Behälter 1 vermindert oder zumindest beschränkt werden. Dabei entspricht der Druck P₁ in dem Einweg-Behälter 1 im Wesentlichen dem Druck P₂ in der Hauptleitung 3a, sofern kein wesentlicher Widerstand zwischen dem Einweg-Behälter 1 und der Hauptleitung 3a vorliegt.

Auch in dieser Ausführungsform kann es eine Rückkopplung, wie sie in Fig. 3 gezeigt ist, zwischen der Überdrucksicherung 5 und dem Druck erzeugenden Mittel 4 geben, wobei das Druck erzeugende Mittel 4 mittels eines Signals der Rückkopplung abgeschaltet werden kann. Eine solche Rückkopplung kann in dem gezeigten System 100 optional integriert sein, was jedoch nicht zwingend der Fall sein muss. Die Rücckopplung ist in dieser Darstellung nicht gezeigt.

Die gezeigten die Zu- und/oder Abläufe 2 können in einem geöffneten und/oder in einem geschlossenen Zustand vorliegen. Jeder Zu- und/oder Ablauf 2 kann also entweder offen oder geschlossen sein. Insbesondere kann ein die Zu- und/oder Ablauf 2 geöffnet werden, um einen Druck P, beispielsweise einen Druck P₁ in dem Einweg-Behälter 1 an den Außendruck anzugleichen und/oder um diesen Druck P₁ zu vermindern.

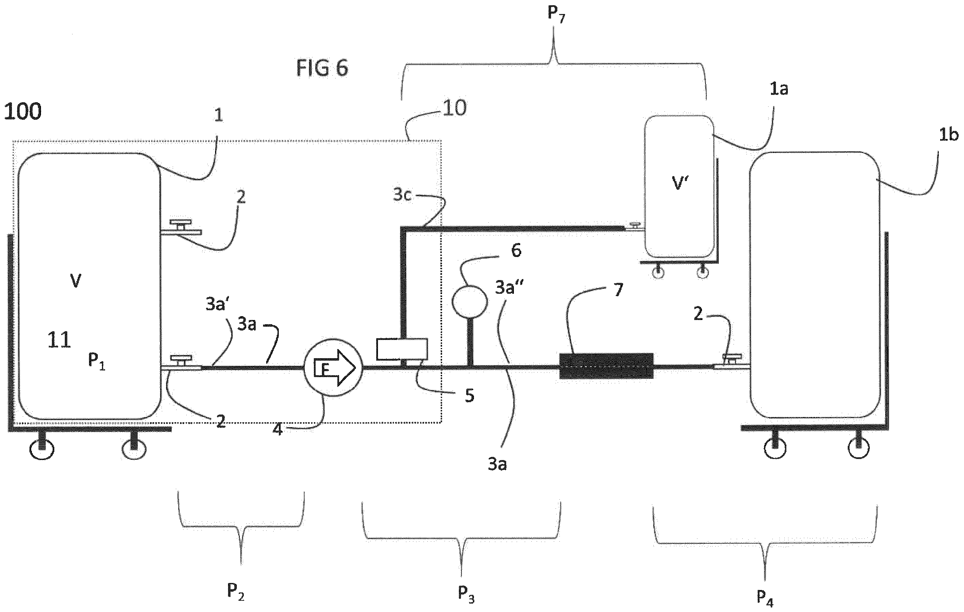

Die **Fig. 6** zeigt ebenfalls ein System 100 gemäß einer weiteren Ausführungsform, welches eine Vorrichtung 10 umfasst. Das System umfasst einen Einweg-Behälter 1, einen zweiten Behälter 1a, der als Auffangbehälter verstanden werden kann und einen dritten Behälter 1b, in den zumindest ein Teil des zumindest einen Mediums 11 im Normalbetrieb, also ohne Überschreiten des Auslösedrucks der Überdrucksicherung 5, befördert werden soll.

Sobald der spezielle (vorbestimmte bzw. vorbestimmbare) Auslösedruck der Überdrucksicherung 5 an dessen unmittelbarer Umgebung überschritten wird, löst die Überdrucksicherung 5 aus und so wird zumindest ein Teil des zumindest einen Mediums 11 durch die Ableitung 3c dem Volumen V' des zweiten Behälters 1a zugeführt, sofern der Druck P₇ in der Ableitung und dem zweiten Behälter 1a nicht den Auslösedruck, der hier beim Auslösen der Überdrucksicherung 5 dem Druck P₃ entspricht, übersteigt.

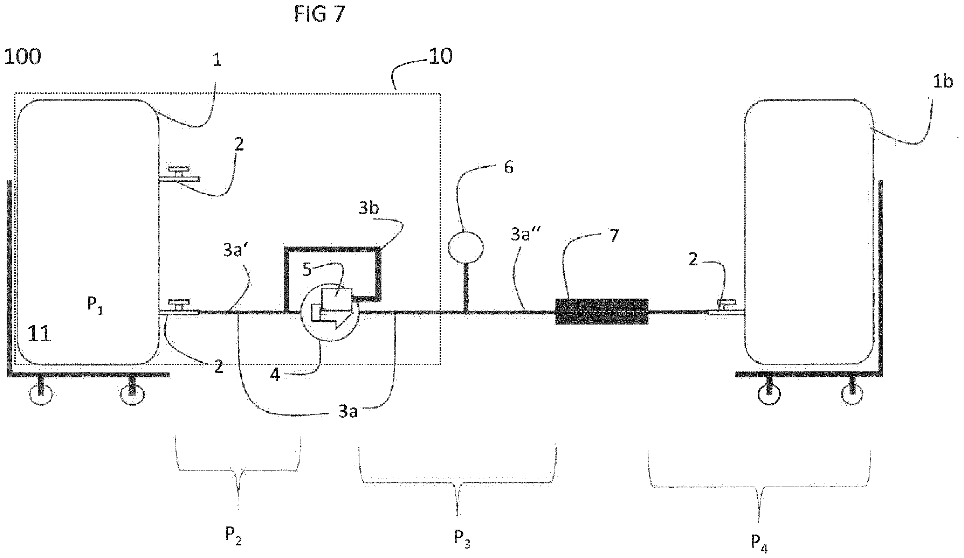

Die **Fig. 7** zeigt ebenfalls ein System 100 gemäß einer weiteren Ausführungsform, welches eine Vorrichtung 10 umfasst. An einer Hauptleitung 3a ist ein Druck erzeugendes Mittel 4 angeordnet, welches eine interne bzw. integrierte Überdrucksicherung 5 umfasst. Ferner umfasst das System 100 eine Bypass-Leitung 3b. Sobald der Druck P₃ einen speziellen (vorbestimmten bzw. vorbestimmbaren) Auslösedruck der Überdrucksicherung 5 überschreitet, wird die Überdrucksicherung 5 ausgelöst und leitet zumindest einen Teil des zumindest einen Mediums 11 in die Bypass-Leitung 3b ab. Die Bypass-Leitung 3b ist dabei ausgelegt, das entsprechende Medium 11 in einen - bezüglich der Fluss- bzw. Stromrichtung F in der Hauptleitung 3a - der Überdrucksicherung 5 vorgelagerten Abschnitt 3a' des Systems 100, und zwar der Hauptleitung 3a zurückzuführen. Dies ist dann möglich, wenn der Druck P₂ vor der Pumpe 4 einen niedrigeren Wert aufweist als der Druck P₃ zwischen Pumpe 4 und Filter 7. Auf diese Weise kann der Druck P₃ in einem - bezüglich der Stromrichtung F in der Hauptleitung 3a - der Überdrucksicherung 5 nachgelagerten Abschnitt 3a" des Systems 100, und zwar der Hauptleitung 3a vermindert oder zumindest beschränkt werden.

Die Bypass-Leitung 3b kann alternativ auch in das Druck erzeugende Mittel 4 bzw. in die Pumpe 4 vollständig oder zumindest teilweise integriert sein. Zumindest kann die Pumpe 4 einen Anschluss für eine Bypass-Leitung 3b aufweisen.

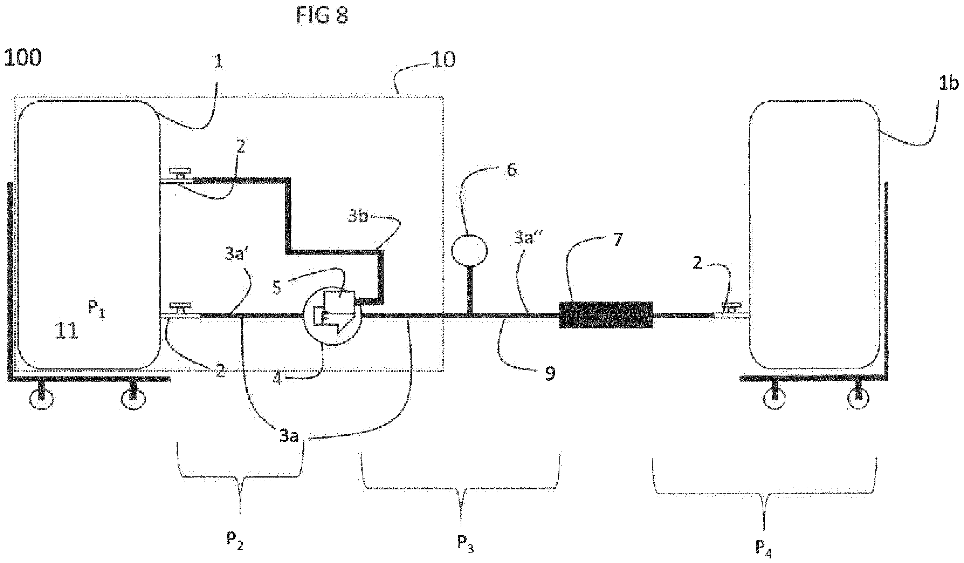

Die **Fig. 8** zeigt ebenfalls ein System 100 gemäß einer weiteren Ausführungsform, welches eine Vorrichtung 10 umfasst. An einer Hauptleitung 3a ist ein Druck erzeugendes Mittel 4 bzw. eine Pumpe 4 angeordnet, welches bzw. welche eine interne bzw. integrierte Überdrucksicherung 5 umfasst. Ferner umfasst das System 100 auch eine Bypass-Leitung 3b. Sobald der Druck P₃ einen speziellen (vorbestimmten bzw. vorbestimmbaren) Auslösedruck der Überdrucksicherung 5 überschreitet, wird die Überdrucksicherung 5 ausgelöst und leitet zumindest einen Teil des zumindest einen Mediums 11 in die Bypass-Leitung 3b ab. Die Bypass-Leitung 3b ist dabei ausgelegt, das entsprechende Medium 11 in einen - bezüglich der Fluss- bzw. Stromrichtung F in der Hauptleitung 3a - der Überdrucksicherung 5 vorgelagerten Abschnitt 3a' des Systems 100, und zwar in den Einweg-Behälter 1 zurückzuführen.

Die Bypass-Leitung 3b kann auch in diesem Fall zumindest teilweise in dem Druck erzeugenden Mittel 4 bzw. die Pumpe 4 integriert sein. Zumindest kann die Pumpe 4 einen Anschluss für eine Bypass-Leitung 3b aufweisen.

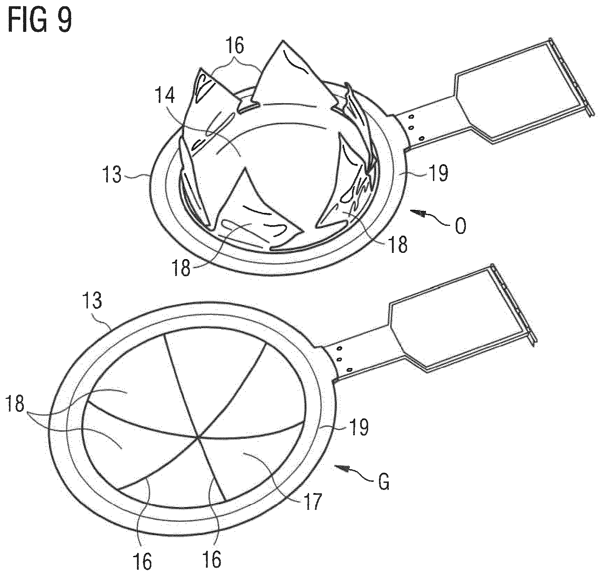

Die **Fig. 9** zeigt eine weitere Ausführungsform einer Überdrucksicherung 5, und zwar eine Berstscheibe 13, welche in einem intakten geschlossenen Zustand G und in einem zerborstenen bzw. ausgelösten bzw. offenen Zustand O vorliegt. Die Überdrucksicherung 5 ist zumindest teilweise aus einem Metall, insbesondere einem Aluminium und/oder einem Edelstahl ausgebildet. Alternativ kann die Berstscheibe 13 auch aus einem Kunststoff ausgebildet sein, insbesondere dann, wenn eine Korrosion des Materials verhindert werden soll.

In dem geschlossenen Zustand G sind ein oder mehrere Sollbruchstellen 16 bzw. Nähte auf der geschlossenen Fläche der Berstscheibe zu erkennen. Die konvex gewölbte Fläche 17 ist in sechs dreieckige Teilabschnitte 18 der konvex gewölbten Fläche 17 der Berstscheibe 13 unterteilt, welche jeweils durch eine Sollbruchstelle 16 voneinander getrennt werden. Die Sollbruchstellen 16 sind dabei symmetrisch innerhalb eines ringförmigen Rahmens 19 angeordnet.

Im offenen Zustand O, der dem Zustand entspricht, wenn die Berstscheibe 13 ausgelöst wurde, werden die dreieckigen Teilabschnitte 18 der konvex gewölbten Fläche 17 der Berstscheibe 13 entlang der Sollbruchstellen 16 jeweils voneinander gelöst bzw. abgelöst, sodass diese lediglich von einem ringförmigen Rahmen 19 gehalten werden und im Wesentlichen eine Öffnung 14 bilden.

Bei dem Auslösen (insbesondere beim Erreichen des speziellen (vorbestimmten bzw. vorbestimmbaren) Auslösedrucks) zerberstet bzw. bricht bzw. öffnet die Berstscheibe 13 entlang der Sollbruchstellen 16 fragmentfrei, sodass kein Partikel und/oder Element der Berstscheibe 13 in das System 100 und/oder das Medium 11 gelangt. In der Regel weist eine Berstscheibe 13 einen Auslösedruck auf. Die Berstscheibe 13 ist entsprechend so ausgestaltet, dass sie zerberstet wenn dieser Auslösedruck überschritten wird. Die kann beispielsweise durch die Wahl des Materials und/oder die Stabilität der Sollbruchstellen 16 und/oder die Wölbung der Berstscheibe 13 vorbestimmt bzw. definiert werden.

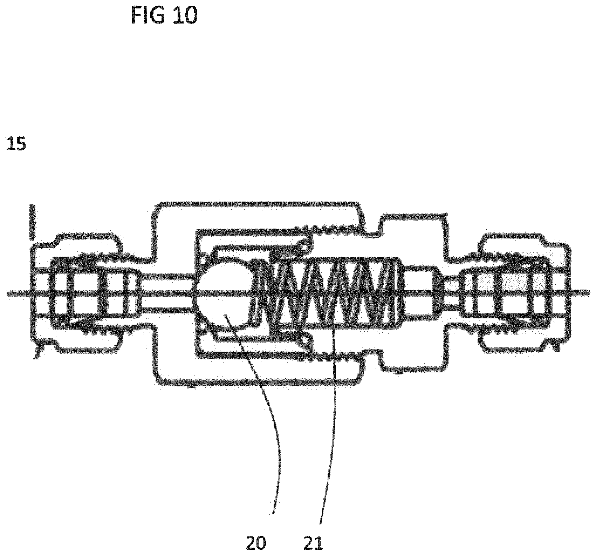

Die **Fig. 10** zeigt eine weitere Ausführungsform einer Überdrucksicherung 5, und zwar ein Rückschlagventil 15 gemäß einer Ausführungsform. Ein Rückschlagventil 15 bzw. eine Rückschlagarmatur ist ein Bauteil, welches die Strömung eines Fluids in lediglich eine Richtung gewährt. Die **Fig. 10** zeigt ein federbelasteten Rückschlagventil 15, bei dem ein Schließelement 20 in einer Richtung durch eine Rückstell-Feder 21 geschlossen wird und in der anderen Richtung dagegen vom Druck eines strömenden Fluids freigegeben wird. Hierbei wird insbesondere eine Kugel als Schließelement 20 verwendet. Alternativ kann ein Kegel, eine Klappe oder eine Membran in den jeweiligen Sitz gedrückt werden. Steht in der Durchlassrichtung ein Druck an, der die Kraft der Rückstell-Feder 21 überwinden kann, so wird das Schließelement 20 von seinem Sitz getrennt bzw. abgehoben und der Durchfluss wird gewährt. Andere Ausführungsformen kommen auch ohne Feder aus, wobei das Schließelement 20 nur durch das strömende Fluid öffnen und insbesondere durch die Gewichtskraft des Schließelements 20 schließen kann.

Es versteht sich von selbst, dass die in **Fig. 9** und **10** gezeigten Überdrucksicherungen 5 in einer der Vorrichtungen 100 und/oder einem der Systeme 10 und/oder einem der Einweg-Behälter 1 und/oder Behälter 1a, 1b integriert sein können bzw. davon umfasst bzw. damit verbunden sein können.

Die zuvor beschriebenen Ausführungsformen der Vorrichtung 10, des Systems 100, sowie der Methode, können in verschiedenen Bereichen angewandt werden. Insbesondere ist der Bereich der Biotechnologie betroffen, jedoch können auch andere Anwendungsbereiche in Betracht kommen, wie beispielsweise: Lebensmitteltechnologie, Getränketechnologie, chemische Industrie, chemische Forschung, Laborbedarf, Medizintechnik (zum Beispiel Zubehör für Blutkonserven und/oder Dialysen und/oder Infusionen), Prozesschemie, technische Chemie.

Folgender bzw. folgende Prozesse können unter anderem innerhalb eines Behälters stattfinden: chemische und/oder biologische und/oder biochemische und/oder physikalische Prozesse, insbesondere Fermentation, Vergärung, Destillation, Aufreinigung, Zersetzung, aerobe Prozesse, anaerobe Prozesse. Insbesondere können Vorrichtung 10, System 100, und Methode in Verbindung mit FlexAct Systemen, Sartoflow Crossflow-Anlagen, Chromatographianlagen und Anlagen zur Zellernte, sowie single-use bzw. Einweg-Bioreaktoren zum Einsatz kommen.

Die Vorrichtung 10, sowie deren Ausführungsformen können beispielsweise auch in der Lagerung und/oder Verarbeitung gefährlicher und/oder toxischer und/oder explosiver Chemikalien Anwendung finden. Beispielsweise kann eine Flasche, in der ein explosiver und/oder toxischer Stoff eines Mediums gelagert und/oder transportiert wird, eine Überdrucksicherung umfassen, die ausgelegt ist, zumindest einen Teil des Mediums im Falle eines Überdrucks in ein anderes Gefäß bzw. einen anderen Behälter zu leiten. Ein anderer Fall könnte einen Abfallkanister für Lösemittel betreffen, in dem sich oft gefährliche und/oder reaktive Lösemittelgemische befinden können. Ein solcher Abfallkanister oder eine Flasche, wie sie zuvor genannt wurde, kann bzw. können beispielsweise als Einweg-Behälter betrachtet werden und Teil der erfindungsgemäßen Vorrichtung sein. Dies hätte den Vorteil, ein unkontrolliertes Austreten zumindest eines der genannten Medien und/oder der genannten Stoffe, oder gar das Zerbersten des Kanisters und/oder der Flasche verhindert werden kann.

In anderen Worten, hat ein Einweg-Element, wie ein Einweg-Behälter 1, insbesondere ein Einweg-Bioreaktor den Vorteil, dass dieser steril bereitgestellt werden kann und nach der Verwendung und Kontamination mit Inhalt nicht wieder gereinigt bzw. autoklaviert werden muss, sondern entsorgt werden kann. Durch die Verwendung kostengünstiger Materialien zur Herstellung von Einweg-Bioreaktoren können Prozesse besonders kostengünstig durchgeführt bzw. umgesetzt werden. Es können alle Bestandteile einer Vorrichtung, eines Systems, sowie eines Behälters, insbesondere eines Bioreaktors, sowie sämtliches Zubehör als Einweg-Elemente ausgebildet sein. Alternativ können lediglich einzelne Bestandteile einer Vorrichtung, eines Systems, sowie eines Behälters, insbesondere eines Bioreaktors als Einweg-Elemente ausgebildet sein, wohingegen andere Bestandteile Mehrweg-Elemente darstellen.

Als Medium 11 bzw. Medien werden im Rahmen der vorliegenden Erfindung insbesondere Flüssigkeiten, Gase, Suspensionen, Dispersionen, Puffer und/oder Zellkulturbrühen angesehen. Medien können ferner auch Feststoffe, wie beispielsweise Pulver, gepresste Pellets, Partikel, Körner, sowie Mischungen daraus umfassen. Ein Medium kann entsprechend unterschiedliche Bestandteile mit gleichem oder unterschiedlichem Aggregatszustand, beispielsweise eine Emulsion oder eine Dispersion umfassen.

Unter Behältern werden im Rahmen der vorliegenden Erfindung insbesondere Einweg-Behälter 1 verstanden. Bevorzugt handelt es sich um Einweg- bzw. Single-use-Beutel aus einem weichen Kunststoff. Behälter können einen oder mehrere aus den Folgenden umfassen: Behälter zum Mischen, Lagern und/oder Transportieren, sowie Bioreaktoren bzw. Behälter als Bestandteil von Bioreaktoren und Fermentern, aber auch Gefäße, Kanister und Behälter zur Lagerung von Medien und/oder Pufferlösungen. Ein Bioreaktor oder Fermenter kann einen Behälter umfassen oder darstellen. Es kann sich ferner bei dem Behälter auch beispielsweise um einen Mischtank bzw. -behälter, einen Lagerbehälter, eine Flasche, einen Kanister oder einen Lebensmitteltank bzw. -fass handeln. Es kann sich bei dem Behälter auch um Behälter handeln, in denen chemisches Material aufbewahrt, transportiert und/oder prozessiert wird, auch kann ein Behälter ein chemisches Laborgerät, beispielsweise ein Säulengefäß für die Säulenchromatographie, ein Gefäß oder ein Element beispielsweise einer Destille darstellen. Ein Behälter kann insbesondere zumindest teilweise aus Kunststoff ausgebildet sein. Alternativ kann ein Behälter auch zumindest teilweise aus einem Metall insbesondere aus Stahl ausgebildet sein.

Ferner kann ein Behälter zumindest teilweise aus Glas ausgebildet sein. Es sei ausdrücklich erwähnt, dass die genannten Behälter auch Mehrweg-Behälter sein können. Beispielsweise kann ein Auffangbehälter auch ein Kanister sein, der zur mehrfachen Verwendung ausgelegt sein kann.

Behälter, wie Bioreaktoren, Mischsysteme und Pellet-Tanks dienen im Wesentlichen zur Aufnahme, zur Lagerung und/oder zum Mischen von biologischen Medien, wie z.B. Fluiden und/oder Feststoffen und/oder Gasen. Biologische Medien können in Behältern, wie z.B. Beuteln, insbesondere in Kunststoffbeuteln bereitgestellt werden, die ein Volumen von mehreren hundert oder sogar mehreren tausend Litern umfassen können. Die biologischen Medien können bevorzugt innerhalb eines solchen Beutels in den Bioreaktor eingebracht werden, in dem sie gelagert, temperiert und/oder durchmischt werden können.

Einweg-Behälter 1 können insbesondere in Filtrations-/ Fermentations- und Aufarbeitungsanlagen, wie beispielsweise Virusfiltration-/ Chromatographie- oder Crossflowanlagen integriert sein.

Der Einweg-Behälter 1 kann ein Einweg-Bioreaktorbeutel mit einer flexiblen Wandung sein, wie beispielsweise ein Biostat^{®} Cultibag^{®}-Rührreaktor, -Bioreaktorbeutel, ein Cultibag^{®} RM-Rocking-Bioreaktorbeutel vom Wave^{®}-Typ, ein Schüttelbeutel vom Schüttel-ORB^{®}-Typ oder andere flexible Bioreaktortaschen. Bei dem Einweg-Behälter 1 kann es sich ferner um eine Einweg-3D-Bioprocessing-Tasche, beispielsweise eine Palletank^{®}-Aufbewahrungs- oder Mischtasche, eine LevMix^{®}- oder MagMix^{®}-Mischtasche, andere flexible 3D-Bioprocessing-Beutel oder 3D-Beutelumhüllungen handeln. Bei dem Einweg-Behälter 1 kann es sich auch um einen Einweg-Bioprozessbeutel aus 2D, z. B. um einen FlexBoy^{®}, Celsius^{®} oder andere 2D-Bioprozessbeutel handeln. Der Einweg-Behälter 1 kann auch ein Einweg-Bioreaktorbehälter mit einer starren Behälterwandung 12 sein, wie beispielsweise der Biostat^{®} SU, der TAP Biosystems ambr15, der ambr250 und andere starre ummantelte Bioreaktorbehälter. Der Einweg-Behälter 1 kann auch ein starrwandiger CellSTACK^{®}-Behälter für den Einmalgebrauch, der ein Mehrkammer-Kunststoffbehälter mit starren Wänden ist, sein. Es werden beispielsweise für die Gewebekultur von wachsenden adhärenten Zellen unter Anderem festwandige CellSTACK ^{®} Behälter verwendet.

Mit dem Begriff "Auslösen einer Überdrucksicherung 5" ist insbesondere gemeint, dass die Überdrucksicherung 5 in dem Moment des Auslösens ihrer Funktion nachkommt. Insbesondere kann ein Auslösen darin bestehen, dass ein Druck einen Maximalwert, und zwar einen Auslösedruck überschreitet, wodurch eine Überdrucksicherung 5 derart ausgelöst wird, dass sie eine Öffnung bildet und somit zumindest ein weiteres Ansteigen des Drucks P innerhalb der Vorrichtung 10 oder eines Abschnitts der Vorrichtung 10 verhindert werden kann. Durch die Öffnung kann nach der Auslösung der Überdrucksicherung 5 zumindest ein Teil des in der Vorrichtung 10 befindlichen Mediums 11 entweichen und/oder kontrolliert austreten und/oder ab- und/oder zurückgeführt werden. In besonderen Fällen kann nach Auslösung einer Überdrucksicherung 5 der Druck P in der Vorrichtung 10 oder in zumindest einem Abschnitt der Vorrichtung gegenüber dem Wert vor der Auslösung sogar gesenkt werden.

Eine Überdrucksicherung 5 kann auch fluidisch mit zumindest einem Filter verbunden sein. Ein solcher Filter kann, ohne darauf beschränkt zu sein, ein hydrophober Entlüftungsfilter, ein hydrophiler Flüssigkeitsfilter, ein sterilisierender Filter, ein Virus-Rückhaltefilter, ein kombinierter hydrophiler/hydrophober Filter, ein Sperrfilter, ein Filterzug mit mehreren Filtern sein bzw. umfassen. Filter können verhindern, dass toxische und/oder gefährliche Stoffe, insbesondere Aerosole, die aus dem System 100 und/oder der Vorrichtung 10 treten, einen Verwender beeinträchtigen und/oder in die Umgebung entweichen.

Eine Leitung 3, eine Bypass-Leitung 3b, eine Ableitung 3c und/oder eine Überdrucksicherung 5 kann mindestens einen Filter, insbesondere einen Membranfilter umfassen. Der Filter kann ein hydrophober Entlüftungsfilter, ein hydrophiler Flüssigkeitsfilter, ein sterilisierender Filter, ein Virus-Rückhaltefilter, ein Kombinations-Hydrophil/Hydrophob-Filter, ein Sperrfilter und/oder ein Filterstrang mit mehreren Filtern sein, ohne darauf beschränkt zu sein. Es kann auch eine Abzweigung bzw. eine Kombination von Filtern vorliegen, die separate hydrophile und hydrophobe Filter einschließen können.

Eine Vorrichtung 10 umfasst mindestens die vom ersten Hauptanspruch umfassten Merkmale der Vorrichtung 10. Die in den Zeichnungen bzw. Figuren beispielhaft dargestellten Ausführungsformen der Vorrichtung 10 umfassen auch zusätzliche Merkmale, die optional zu verstehen sind.

Ein Ziel-Behälter umfasst alle genannten Behälter, die als Einweg-Behälter in Frage kommen. Darüber hinaus umfasst ein Ziel-Behälter ein offenes oder geschlossenes Gefäß, eine (Rohr-)Leitung, einen Container, einen (Bio-)Reaktor und sonstige Behältnisse zur Aufbereitung oder Prozessierung von Fluiden.

### Bezugszeichen

- 1: Einweg-Behälter
- 1a: Weiterer zweiter Behälter
- 1b: Weiterer dritter Behälter auch Ziel-Behälter genannt
- 2: Zu- und/oder Ablauf
- 3: Leitung
- 3a: Hauptleitung
- 3a': Bezüglich einer Stromrichtung in der Hauptleitung vor Überdrucksicherung vorgelagerter Abschnitt
- 3a": Bezüglich einer Stromrichtung in der Hauptleitung nach Überdrucksicherung nachgelagerter Abschnitt
- 3b: Bypass-Leitung
- 3c: Ableitung
- 4: Druck erzeugendes Mittel, beispielsweise Pumpe
- 5: Überdrucksicherung
- 6: Druckmessgerät, beispielsweise Manometer
- 7: Widerstand, beispielsweise Filter(anlage)
- 8: Rückkopplung zwischen Überdrucksicherung und Pumpe
- 9: System, das offen oder geschlossen sein kann
- 10: Vorrichtung
- 10a: Erstes Teilsystem, das geschlossen oder offen ist
- 10b: Zweites Teilsystem, das geschlossen oder offen ist
- 11: Medium
- 12: Behälterwandung
- 13: Berstscheibe
- 14: Öffnung in der Berstscheibe
- 15: Rückschlagventil
- 16: Sollbruchstellen bzw. Naht
- 17: Konvex gewölbte Fläche einer Berstscheibe
- 18: Dreieckige Teilabschnitte der konvex gewölbten Fläche einer Berstscheibe
- 19: Ringförmiger Rahmen
- 20: Schließelement eines Rückschlagventils
- 21: Rückstellfeder eines Rückschlagventils
- 100: System
- F: Flussrichtung
- G: Geschlossener Zustand der Überdrucksicherung
- O: Offener Zustand der Überdrucksicherung
- P: Druck vor Überdrucksicherung
- P₁: Druck im Behälter
- P₂: Druck in Hauptleitung vor Pumpe
- P₃: Druck in Hauptleitung nach Pumpe
- P₄: Druck in Hauptleitung und ggf. In zweitem Behältnis nach Wiederstand
- P₅: Druck vor Druck beaufschlagtem Einweg-Behälter
- P₆: Druck in Ableitung und ggf. in drittem Behältnis
- V: Behältervolumen des Einweg-Behälters
- ZV: Zielvolumen

## Patentansprüche

1. System (100) zum Lagern und/oder Verarbeiten mindestens eines Mediums (11), insbesondere Bioprozesseinrichtung, umfassend
- mindestens einen Einweg-Behälter (1), der ausgelegt ist, das mindestens eine Medium (11) zumindest teilweise aufzunehmen; und
- mindestens eine Überdrucksicherung (5), welche fluidisch mit dem mindestens einem Einweg-Behälter (1) verbunden ist, wobei die mindestens eine Überdrucksicherung (5) ausgelegt ist, bei einer Auslösung, das mindestens eine Medium (11) zumindest teilweise
- in einen bezüglich einer Stromrichtung (F) der Überdrucksicherung (5) vorgelagerten Vorrichtungsbereich (3a') zu führen, und/oder
- in den mindestens einen Einweg-Behälter (1) zu führen; und/oder
- in mindestens einen weiteren zweiten Behälter (1a) zu führen,
wobei das System ferner umfasst:
- eine Hauptleitung (3a) zur fluidischen Verbindung des mindestens einen Einweg-Behälters (1) mit einem Ziel-Behälter (1b);
- mindestens eine Ableitung (3c), die ausgelegt ist, bei der Auslösung der mindestens einen Überdrucksicherung (5) das mindestens eine Medium (11) zumindest teilweise in mindestens einen weiteren Behälter (1a) zu leiten; und/oder
- eine Bypass-Leitung (3b), die ausgelegt ist, bei der Auslösung der mindestens einen Überdrucksicherung (5) das mindestens eine Medium (11) zumindest teilweise in den bezüglich einer Stromrichtung (F) der Überdrucksicherung (5) vorgelagerten Vorrichtungsbereich (3a'), insbesondere einen Abschnitt der Hauptleitung und/oder in den mindestens einen Einweg-Behälter (1) zurück zu leiten,
- wobei die mindestens eine Überdrucksicherung (5) an der Ableitung (3c) und/oder der Bypass-Leitung (3b) angeordnet ist und bei der Auslösung der mindestens einen Überdrucksicherung (5) die Ableitung (3c) und/oder die Bypass-Leitung (3b) fluidisch mit der Hauptleitung (3a) verbunden ist,
- mindestens ein Druck erzeugendes Mittel (4), insbesondere eine Pumpe, wobei das mindestens eine Druck erzeugende Mittel (4) ausgelegt ist, zumindest einen Teil des mindestens einen Mediums von dem mindestens einen Einweg-Behälter (1) in den dritten Behälter (1b) entlang der Flussrichtung (F) zu pumpen,
wobei das mindestens eine Druck erzeugende Mittel (4) an dem Einweg-Behälter (1) angeordnet ist und ausgelegt ist, ein Druckgefälle innerhalb des Einweg-Behälters (1) und/oder entlang der Hauptleitung (3a) zu erzeugen.

2. System (100) nach Anspruch 1, wobei die mindestens eine Überdrucksicherung (5) unmittelbar in einer Behälterwandung (12) des mindestens einen Einweg-Behälters (1) integriert ist.

3. System (100) nach einem der vorangehenden Ansprüche, wobei die mindestens eine Überdrucksicherung (5) eine Einweg-Überdrucksicherung, insbesondere eine mechanische Einweg-Überdrucksicherung umfasst, die bevorzugt zumindest teilweise aus einem Edelstahl und/oder einem Kunststoff ausgeformt ist.

4. System (100) nach einem der vorangehenden Ansprüche, wobei die mindestens eine Überdrucksicherung (5) mindestens eine aus den Folgenden umfasst: eine Berstscheibe, ein Überströmventil, ein Sicherheitsventil, eine Membran und insbesondere ein elektrischer und/oder ein mechanischer Kraftbegrenzer einer Pumpe.

5. System (100) nach einem der vorangehenden Ansprüche, ferner umfassend mindestens eine Ableitung (3c), die dazu ausgelegt ist, bei der Auslösung der mindestens einen Überdrucksicherung (5) das mindestens eine Medium (11) zumindest teilweise in den mindestens einen Einweg-Behälter (1) und/oder in mindestens einen zweiten Behälter (1a) zu leiten.

6. System (100) nach einem der vorangehenden Ansprüche, ferner umfassend den Ziel-Behälter (1b), der ausgelegt ist, das mindestens eine Medium (11) zumindest teilweise aufzunehmen und der bevorzugt ein Einweg-Behälter ist.

7. System (100) nach einem der vorangehenden Ansprüche, wobei das mindestens eine Druck erzeugende Mittel (4) an der Hauptleitung (3a) angeordnet ist und ausgelegt ist, ein Druckgefälle entlang der Hauptleitung (3a) zu erzeugen.

8. System (100) nach einem der vorangehenden Ansprüche, wobei die Ableitung (3c) und/oder die Bypass-Leitung (3b) zumindest teilweise in dem Druck erzeugenden Mittel (4) integriert ist bzw. sind.

9. System (100) nach Anspruch 7 oder 8, ferner umfassend eine Rückkopplung (8), insbesondere eine elektrische Rückkopplung, zwischen Überdrucksicherung (5) und Druck erzeugendem Mittel (4), wobei die Rückkopplung (8) ausgelegt ist, bei der Auslösung der mindestens einen Überdrucksicherung (5), das Druck erzeugende Mittel (4) abzuschalten.

10. Verfahren zum zumindest teilweisen Auffangen mindestens eines Mediums (11) in dem System nach einem der vorangehende Ansprüche umfassend die Schritte:
- Bereitstellen mindestens eines Einweg-Behälters (1), der ausgelegt ist, das mindestens eine Medium (11) zumindest teilweise aufzunehmen;
- Bereitstellen mindestens einer Überdrucksicherung (5), welche fluidisch mit dem mindestens einem Einweg-Behälter (1) verbunden ist; und
für den Fall, dass die Überdrucksicherung (5) ausgelöst wird:
- Ableiten des mindestens einen Mediums (11) zumindest teilweise in einen weiteren zweiten Behälter (1a) mittels einer Ableitung (3c); und/oder
- Rückführen des mindestens einen Mediums (11) zumindest teilweise in einen bezüglich einer Stromrichtung (F) der Überdrucksicherung (5) vorgelagerten Vorrichtungsbereich (3a'), insbesondere in den mindestens einen Einweg-Behälter (1), mittels einer Bypass-Leitung (3b).

## Claims

1. System (100) for storing and/or processing at least one medium (11), in particular a bioprocess device, comprising:
- at least one disposable container (1) which is configured to at least partially receive the at least one medium (11); and
- at least one overpressure protection means (5) which is connected to the at least one disposable container (1) in fluid terms, wherein the at least one overpressure protection means (5) is configured, in the event of an activation, to guide the at least one medium (11) at least partially
- into a device region (3a') which is arranged upstream of the overpressure protection means (5) with respect to a flow direction (F), and/or
- into the at least one disposable container (1); and/or
- into at least one additional second container (1a),
wherein the system further comprises:
- a main line (3a) for fluid connection of the at least one disposable container (1) to a target container (1b) ;
- at least one discharge (3c) which is configured, when the at least one overpressure protection means (5) is activated, to direct the at least one medium (11) at least partially into at least one additional container (1a); and/or
- a bypass line (3b) which is configured, when the at least one overpressure protection means (5) is activated, to direct the at least one medium (11) at least partially back into the device region (3a') which is arranged upstream of the overpressure protection means (5) with respect to a flow direction (F), in particular into a portion of the main line and/or into the at least one disposable container (1),
- wherein the at least one overpressure protection means (5) is arranged on the discharge (3c) and/or the bypass line (3b) and, when the at least one overpressure protection means (5) is activated, the discharge (3c) and/or the bypass line (3b) is/are connected in fluid terms to the main line (3a),
- at least one pressure-producing means (4), in particular a pump, wherein the at least one pressure-producing means (4) is configured to pump at least a portion of the at least one medium from the at least one disposable container (1) into the third container (1b) in the flow direction (F),
wherein the at least one pressure-producing means (4) is arranged on the disposable container (1) and is configured to produce a pressure drop within the disposable container (1) and/or along the main line (3a).

2. System (100) according to claim 1, wherein the at least one overpressure protection means (5) is integrated directly in a container wall (12) of the at least one disposable container (1) .

3. System (100) according to any one of the preceding claims, wherein the at least one overpressure protection means (5) comprises a disposable overpressure protection means, in particular a mechanical overpressure protection means, which is preferably at least partially formed from a high-grade steel and/or a plastics material.

4. System (100) according to any one of the preceding claims, wherein the at least one overpressure protection means (5) comprises at least one of the following: a bursting disk, an overflow valve, a safety valve, a membrane and in particular an electrical and/or mechanical force limiter of a pump.

5. System (100) according to any one of the preceding claims, further comprising at least one discharge (3c) which is configured, when the at least one overpressure protection means (5) is activated, to direct the at least one medium (11) at least partially into the at least one disposable container (1) and/or into at least one second container (1a).

6. System (100) according to any one of the preceding claims, further comprising the target container (1b) which is configured to at least partially receive the at least one medium (11) and which is preferably a disposable container.

7. System (100) according to any one of the preceding claims, wherein the at least one pressure-producing means (4) is arranged on the main line (3a) and is configured to produce a pressure drop along the main line (3a).

8. System (100) according to any one of the preceding claims, wherein the discharge (3c) and/or the bypass line (3b) is/are integrated at least partially in the pressure-producing means (4).

9. System (100) according to claim 7 or 8, further comprising a feedback (8), in particular an electrical feedback, between the overpressure protection means (5) and the pressure-producing means (4), wherein the feedback (8) is configured to switch off the pressure-producing means (4) when the at least one overpressure protection means (5) is activated.

10. Method for at least partially collecting at least one medium (11) in the system according to any one of the preceding claims, comprising the steps of:
- providing at least one disposable container (1) which is configured to at least partially receive the at least one medium (11);
- providing at least one overpressure protection means (5) which is connected to the at least one disposable container (1) in fluid terms; and,
in the event that the overpressure protection means (5) is activated:
- discharging the at least one medium (11) at least partially into an additional second container (1a) by means of a discharge (3c); and/or
- returning the at least one medium (11) at least partially into a device region (3a') which is arranged upstream of the overpressure protection means (5) with respect to a flow direction (F), in particular into the at least one disposable container (1), by means of a bypass line (3b).

## Revendications

1. Système (100) de stockage et/ou de préparation d'au moins un milieu (11), en particulier un équipement de bioprocédé, comprenant
- au moins un contenant à usage unique (1) qui est conçu pour recevoir au moins partiellement le au moins un milieu (11); et
- au moins une protection contre la surpression (5), laquelle est fluidiquement reliée avec le au moins un contenant à usage unique (1), dans lequel la au moins une protection contre la surpression (5) est conçue pour, lors d'une libération, guider au moins partiellement le au moins un milieu (11)
- jusque dans une zone de dispositif (3a') montée en amont de la protection contre la surpression (5) par rapport à une direction d'écoulement (F), et/ou
- jusque dans le au moins un contenant à usage unique (1); et/ou
- jusque dans au moins un autre second contenant (1a),
dans lequel le système comprend en outre :
- une conduite principale (3a) pour la liaison fluidique du au moins un contenant à usage unique (1) avec un contenant cible (1b);
- au moins une évacuation (3c) qui est conçue pour, lors de la libération de la au moins une protection contre la surpression (5), conduire au moins partiellement le au moins un milieu (11) jusque dans au moins un autre contenant (1a); et/ou
- une conduite de dérivation (3b) qui est conçue pour, lors de la libération de la au moins une protection contre la surpression (5), ramener au moins partiellement le au moins un milieu (11) jusque dans la zone de dispositif (3a') montée en amont de la protection contre la surpression (5) par rapport à une direction d'écoulement (F), en particulier une section de la conduite principale et/ou jusque dans le au moins un contenant à usage unique (1),
dans lequel la au moins une protection contre la surpression (5) est agencée contre l'évacuation (3c) et/ou la conduite de dérivation (3b) et, lors de la libération de la au moins une protection contre la surpression (5), l'évacuation (3c) et/ou la conduite de dérivation (3b) est fluidiquement reliée avec la conduite principale (3a),
- au moins un moyen produisant une pression (4), en particulier une pompe, dans lequel le au moins un moyen produisant une pression (4) est conçu pour pomper au moins une partie du au moins un milieu depuis le au moins un contenant à usage unique (1) jusque dans le troisième contenant (1b) dans la direction d'écoulement (F),
dans lequel le au moins un moyen produisant une pression (4) est agencé contre le contenant à usage unique (1) et conçu pour produire une chute de pression à l'intérieur du contenant à usage unique (1) et/ou le long de la conduite principale (3a).

2. Système (100) selon la revendication 1, dans lequel la au moins une protection contre la surpression (5) est directement intégrée dans une paroi de contenant (12) du au moins un contenant à usage unique (1).

3. Système (100) selon l'une des revendications précédentes, dans lequel la au moins une protection contre la surpression (5) comprend une protection contre la surpression à usage unique, en particulier une protection contre la surpression à usage unique mécanique, qui est de préférence au moins partiellement formée dans un acier inoxydable et/ou une matière plastique.

4. Système (100) selon l'une des revendications précédentes, dans lequel la au moins une protection contre la surpression (5) comprend au moins l'un de ce qui suit : un disque d'éclatement, une soupape de décharge, une soupape de sécurité, une membrane et en particulier un limiteur de force électrique et/ou un limiteur de force mécanique d'une pompe.

5. Système (100) selon l'une des revendications précédentes, comprenant en outre au moins une évacuation (3c) qui est conçue pour, lors de la libération de la au moins une protection contre la surpression (5), conduire au moins partiellement le au moins un milieu (11) jusque dans le au moins un contenant à usage unique (1) et/ou jusque dans au moins un deuxième contenant (1a).

6. Système (100) selon l'une des revendications précédentes, comprenant en outre le contenant cible (1b) qui est conçu pour recevoir au moins partiellement le au moins un milieu (11) et qui est de préférence un contenant à usage unique.

7. Système (100) selon l'une des revendications précédentes, dans lequel le au moins un moyen produisant une pression (4) est agencé contre la conduite principale (3a) et conçu pour produire une chute de pression le long de la conduite principale (3a).

8. Système (100) selon l'une des revendications précédentes, dans lequel l'évacuation (3c) et/ou la conduite de dérivation (3b) est ou sont au moins partiellement intégrés dans le moyen produisant une pression (4).

9. Système (100) selon la revendication 7 ou 8, comprenant en outre une rétroaction (8), en particulier une rétroaction électrique, entre la protection contre la surpression (5) et le moyen produisant une pression (4), dans lequel la rétroaction (8) est conçue pour, lors de la libération de la au moins une protection contre la surpression (5), éteindre le moyen produisant une pression (4).

10. Procédé pour l'interception au moins partielle d'au moins un milieu (11) dans le système selon l'une des revendications précédentes, comprenant les étapes de :
- fourniture d'au moins un contenant à usage unique (1) qui est conçu pour recevoir au moins partiellement le au moins un milieu (11);
- fourniture d'au moins une protection contre la surpression (5), laquelle est fluidiquement reliée avec le au moins un contenant à usage unique (1); et
dans le cas où la protection contre la surpression (5) est libérée :
- évacuer au moins partiellement le au moins un milieu (11) jusque dans au moins un autre second contenant (1a) au moyen d'une évacuation (3c); et/ou
- ramener au moins partiellement le au moins un milieu (11) jusque dans une zone de dispositif (3a') montée en amont de la protection contre la surpression (5) par rapport à une direction d'écoulement (F), en particulier jusque dans le au moins un contenant à usage unique (1), au moyen d'une conduite de dérivation (3b).
